# EUROPEAN PATENT APPLICATION

(11) **EP 4 361 265 A1**
(43) Date of publication of application: **01.05.2024**
(21) Application number: 22204062.8
(22) Date of filing: 27.10.2022
(51) Int. Cl.: C12N 15/10, C12N 9/22, C12N 15/90

(54) **OPTIMIZATION OF EDITING EFFICACY OF CRISPR NUCLEASES WITH COLLATERAL ACTIVITY**

(71) Applicant: BRAIN Biotech AG, 64673 Zwingenberg (DE)
(72) Inventor: SCHOLZ, Paul, 64673 Zwingenberg (DE); ZUREK, Christian, 64673 Zwingenberg (DE)
(74) Representative: Weickmann & Weickmann PartmbB

(57) **Abstract**

The present invention relates to a method for the genome editing of cells at a target locus by homology directed repair (HDR) within a cell population and concurrently enriching within said cell population the HDR-genome edited cells, wherein the method comprises (A) introducing into the cells within the cell population one or more nucleic acid molecules, said one or more nucleic acid molecules encoding in expressible form (i) a CRISPR nuclease comprising or consisting of (a) an amino acid sequence of any one of SEQ ID NO: 1, 2 or 3; (b) an amino acid sequence being encoded by the nucleotide sequence of SEQ ID NO: 4, 5, 6 or 7; (c) an amino acid sequence being at least 80% identical to the amino acid sequence of (a), or (d) an amino acid sequence being encoded by a nucleic acid sequence being at least 80 % identical to the nucleotide sequence of (b), (ii) a guide RNA comprising (a) a first segment comprising a nucleotide sequence that is complementary to a sequence at the target locus and (b) a second segment that interacts with the CRISPR nuclease of (i), wherein the CRISPR nuclease and/or the guide RNA are encoded by said one or more nucleic acid molecules in a form wherein the expression can be induced or repressed, and a nucleic acid molecule comprising, consisting of, or encoding (iii) a donor template with homology to the target locus; and (B) culturing the cells at least for a time wherein the cells double under growth conditions wherein the target locus within the cells of the cell population becomes modified by homology directed repair (HDR), and (C) inducing after step (B) or repressing in steps (A) and (B) the expression of the CRISPR nuclease and/or the guide RNA within the cells thereby enriching within said cell population the HDR-genome edited cells.

## Description

The present invention relates to a method for the genome editing of cells at a target locus by homology directed repair (HDR) within a cell population and concurrently enriching within said cell population the HDR-genome edited cells, wherein the method comprises (A) introducing into the cells within the cell population one or more nucleic acid molecules, said one or more nucleic acid molecules encoding in expressible form (i) a CRISPR nuclease comprising or consisting of (a) an amino acid sequence of any one of SEQ ID NO: 1, 2 or 3; (b) an amino acid sequence being encoded by the nucleotide sequence of SEQ ID NO: 4, 5, 6 or 7; (c) an amino acid sequence being at least 80% identical to the amino acid sequence of (a), or (d) an amino acid sequence being encoded by a nucleic acid sequence being at least 80 % identical to the nucleotide sequence of (b), (ii) a guide RNA comprising (a) a first segment comprising a nucleotide sequence that is complementary to a sequence at the target locus and (b) a second segment that interacts with the CRISPR nuclease of (i), wherein the CRISPR nuclease and/or the guide RNA are encoded by said one or more nucleic acid molecules in a form wherein the expression can be induced or repressed, and a nucleic acid molecule comprising, consisting of, or encoding (iii) a donor template with homology to the target locus; and (B) culturing the cells at least for a time wherein the cells double under growth conditions wherein the target locus within the cells of the cell population becomes modified by homology directed repair (HDR), and (C) inducing after step (B) or repressing in steps (A) and (B) the expression of the CRISPR nuclease and/or the guide RNA within the cells thereby enriching within said cell population the HDR-genome edited cells.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The genome editing technology CRISPR enables genome editing in a broad range of cells and organisms. The CRISPR-based technique substantially streamlined the targeted gene modification in different prokaryotic and eukaryotic cells. However, depending on the cell type and the targeted DNA region within the genome, CRISPR can be inefficient, resulting in less than one percent of edited mammalian cells.

Successful genome editing with CRISPR (or other programmable nucleases) requires three sequential preconditions: (1) Efficient delivery of the CRISPR-encoding genes into the target cell (transfection/transduction efficiency); (2) efficient expression of the CRISPR-components (CRISPR nuclease and the CRISPR-RNAs); and (3) targeting of the gene of interest (GOI) by CRISPR ribonucleoprotein complexes and repair of the DNA by cell's own repair pathways.

The overall success of genome editing depends on the efficiency of each of these single steps. The frequency of successful editing events within a transfected cell population thus correlates with an efficient gene delivery (step 1), high nuclease expression and the formation of functional ribonucleoprotein complexes (step 2) and finally the introduction of double-strand DNA breaks (DSB) followed by the repair of the DSBs in the cell (step 3).

Several approaches have been developed to isolate the subpopulations of cells that express the nuclease, e.g. CRISPR vectors that enable Fluorescence or Magnetic-Activated Cell Sorting (FACS and MACS). These and other available methods allow the enrichment of either transfected cells or cells that express the CRISPR nuclease, which is necessary but not sufficient to obtain edited cells. This is due to the fact that the expression of the nuclease in a given cell does not necessarily imply the formation of functional CRISPR ribonucleoprotein complexes and the introduction of mutations at the targeted DNA site. Thus, since these FACS or MACS-assisted methods do not allow the immediate enrichment of edited cells, genome editing in particular in hard-to-transfect cells (e.g. human primary cell lines) remains still excessively difficult.

A genome co-editing approach for the enrichment of CRISPR-edited cells involving the genome editing of a gene of interest and LRRC8 genes along with the use of blasticidin as a selection marker is described in WO 2019/202099. A related approach of co-editing using 6-thiogunaine (6TG) as the selection agent and the knock-out of the (hypoxanthine phosphoribosyltransferase) HPRT gene (encoding the hypoxanthine phosphoribosyltransferase) to render cells 6TG-resistant is described in Liao et al, Nucleic Acids Res. 2015;43(20):e134. While these approaches efficiently enrich genome-edited cells they are based on the co-editing of a second gene being and selection marker.

The present invention therefore aims at providing technically improved methods and means which enable the enrichment of CRISPR-edited cells (step 3, above). As a yet further advantage the present invention enables the optimized genomic target-specific depletion of cells within a population of cells by CRISPR nuclease activity by timely orchestrating the genome editing event of a CRISPR nuclease and a guide RNA.

Hence, the present invention relates in a first aspect to a method for the genome editing of cells at a target locus by homology directed repair (HDR) within a cell population and concurrently enriching within said cell population the HDR-genome edited cells, wherein the method comprises (A) introducing into the cells within the cell population one or more nucleic acid molecules, said one or more nucleic acid molecules encoding in expressible form (i) a CRISPR nuclease comprising or consisting of (a) an amino acid sequence of any one of SEQ ID NO: 1, 2 or 3; (b) an amino acid sequence being encoded by the nucleotide sequence of SEQ ID NO: 4, 5, 6 or 7; (c) an amino acid sequence being at least 80% identical to the amino acid sequence of (a), or (d) an amino acid sequence being encoded by a nucleic acid sequence being at least 80 % identical to the nucleotide sequence of (b), (ii) a guide RNA comprising (a) a first segment comprising a nucleotide sequence that is complementary to a sequence at the target locus and (b) a second segment that interacts with the CRISPR nuclease of (i), wherein the CRISPR nuclease and/or the guide RNA are encoded by said one or more nucleic acid molecules in a form wherein the expression can be induced or repressed, and a nucleic acid molecule comprising, consisting of, or encoding (iii) a donor template with homology to the target locus; and (B) culturing the cells at least for a time wherein the cells double under growth conditions wherein the target locus within the cells of the cell population becomes modified by homology directed repair (HDR), and (C) inducing after step (B) or repressing in steps (A) and (B) the expression of the CRISPR nuclease and/or the guide RNA within the cells thereby enriching within said cell population the HDR-genome edited cells.

A cell population designates a group of cells. The cell population may be heterogeneous or homogeneous and is preferably homogeneous. A heterogeneous cell population comprises cells of different origin, e.g. from different species or sources and/or different cell-types of one species or source (e.g. body site). By contrast, a homogeneous cell population only comprises cells from one species or source and preferably only cells of one cell-type or one body site. The cells of a homogeneous cell population may be in different stares of the cell cycle.

Genome editing (also known as genome engineering) is a type of genetic engineering in which a gene of interest is inserted, deleted, modified or replaced in the genome of the cell. In accordance with the first aspect of the invention, the genome editing employs the homology directed recombination (HDR) pathway. As compared to non-homologous end-joining (NHEJ) and HDR is the more accurate mechanism for DSB repair due to the requirement of higher sequence homology between the damaged and intact donor strands of DNA. The process is error-free if the DNA template used for repair is identical to the original DNA sequence at the DSB, or it can introduce specific mutations into the damaged DNA. It is therefore to be understood that cells in the population of cells harboring the target locus to be genome edited are capable of HDR.

Genome editing may result in a loss-of-function mutation or a gain-of-function mutation in the genome of the cell. A loss-of-function mutation (also called inactivating mutation) results in the gene of interest having reduced or no function (being partially or wholly inactivated). When the allele has a complete loss of function (wholly inactivated) this is also called herein a (gene) knock-out. Genome editing of the gene of interest is preferably a knock-out. A gene knock-out may be achieved by inserting, deleting, modifying or replacing one or more nucleotides of a gene. A gain-of-function mutation (also called activating mutation) may change the gene of interest such that its effect gets stronger (enhanced activation) or even is superseded by a different (e.g. abnormal) function. A gain-of-function mutation may also introduce a new function or effect into a cell which the cell did not have before. In this context the new gene may be added to the genome of the cell (insertion) or may replace a gene within the genome. A gain-of-function mutation introducing such a new function or effect is also called gene knock-in.

Genome editing via HDR in accordance with the first aspect of the invention relies on (i) a nuclease of the clustered regularly interspaced short palindromic repeats (CRISPR/Cas) system which is also designated "CRISPR nuclease" herein, (ii) a guide RNA that mediates the interaction between CRISPR nucleases and the target locus which is also referred to herein as "spacer / protospacer" (noting that the spacer binds to the target locus while the protospacer (or protospacer adjacent motif (PAM)) binds to the CRISPR nuclease), and (iii) a donor template with homology to the target locus which is also referred to herein as "HDR template".

CRISPR nucleases (or CRISPR-Cas nucleases or Cas nucleases) are a specific type of programmable nucleases. The CRISPR nucleases to be used in accordance with the present invention are so-called BRAIN Engineered Cas proteins (BEC) nucleases. In more detail, the amino acid sequence of SEQ ID NO: 1, 2 or 3 and the nucleotide sequence of SEQ ID NO: 4, 5 or 6 are the amino acid sequences and the nucleotide sequences of the class 2, type V RNA-guided DNA nucleases of BEC85, BEC67 and BEC10 as disclosed in the international application PCT/EP2021/000081. Among the three BEC nucleases BEC10 is most preferred. SEQ ID NO: 7 is BEC10 that has been optimized for the expression in *E. coli.* SEQ ID NO: 7 can therefore also be designated E.coliBEC10.

The CRISPR-Cas system has been harnessed for genome editing in prokaryotes and eukaryotes. A small piece of RNA with a short "guide" sequence that attaches (binds) to a specific target sequence of DNA in a genome is created (the so-called guide RNA (gRNA) or single guide (sgRNA)). The genomic target site of the gRNA can be any ~20 nucleotide DNA sequence, provided it meets two conditions: (i) The sequence is unique compared to the rest of the genome, and (ii) the target is present immediately adjacent to a protospacer adjacent motif (PAM). The PAM sequence is essential for target binding, but the exact sequence depends on which CRISPR nuclease is used. CRISPR nucleases and their respective PAM sequences are known in the art (see https://www.addgene.org/crispr/guide/#pam-table). Hence, the gRNA also binds to the CRISPR nuclease (the BEC enzyme). As in bacteria, the gRNA is used to recognize the target DNA sequence, and the CRISPR nuclease cuts the DNA at the targeted location. Once the DNA is cut, the cell's own DNA repair machinery (NHEJ or HDR) adds or deletes pieces of genetic material, or makes changes to the DNA by replacing an existing segment with a customized DNA sequence. Hence, in the CRISPR-Cas system, the CRISPR nuclease makes a double-stranded break in DNA at a site determined by the short (∼20 nucleotide) gRNA which break is then repaired within the cell by NHEJ or HDR. The CRISPR-Cas system can be multiplexed by adding multiple gRNAs. It was demonstrated that, for example, five different simultaneous mutations can be introduced into mouse embryonic stem cells by using five different gRNA molecules and one CRISPR nuclease.

The designs and structures of donor templates being suitable for HDR are known in the art. HDR is error-free if the repair template is identical to the original DNA sequence at the double-strand break (DSB), or it can introduce very specific mutations into DNA. The three central steps of the HDR pathways are: (1) The 5'-ended DNA strand is resected at the break to create a 3' overhang. This will serve as both a substrate for proteins required for strand invasion and a primer for DNA repair synthesis. (2) The invasive strand can then displace one strand of the homologous DNA duplex and pair with the other. This results in the formation of the hybrid DNA, referred to as the displacement loop (D loop). (3) The recombination intermediates can then be resolved to complete the DNA repair process.

HDR templates used, for example, to introduce mutations or insert new nucleotides or nucleotide sequences into a gene require a certain amount of homology surrounding the target sequence that will be modified. Homology arms can be used that start at the CRISPR-induced DSB. In general, the insertion sites of the modification should be very close to the DSB, ideally less than 10 bp away, if possible. One important point to note is that the CRISPR enzymes may continue to cleave DNA once a DSB is introduced and repaired. As long as the gRNA target site/PAM site remain intact, the CRISPR nuclease will keep cutting and repairing the DNA. This repeated editing may be problematic if a very specific mutation or sequence is to be introduced into a gene of interest. To get around this, the repair template can be designed in such a way that it will ultimately block further CRISPR nuclease targeting after the initial DSB is repaired. Two common ways to block further editing are mutating the PAM sequence or the gRNA seed sequence. When designing a repair template, the size of the intended edit is to be taken into consideration. ssDNA templates (also referred to as ssODNs) are commonly used for smaller modifications. Small insertions/edits may require as little as 30-50 bases for each homology arm, and the best exact number may vary based on the gene of interest. 50-80 base homology arms are commonly used. For example, Richardson et al. (Nat Biotechnol. 2016 Mar; 34(3):339-44) found that asymmetric homology arms (36 bases distal to the PAM and 91 bases proximal to the PAM) supported HDR efficiencies up to 60%. Due to difficulties that might be associated with creating ssODNs longer than 200 bases, it is preferred to use dsDNA plasmid repair templates for larger insertions such as fluorescent proteins or selection cassettes into a gene of interest. These templates can have homology arms of at least 800 bp. To increase the frequency of HDR edits based on plasmid repair templates, self-cleaving plasmids can be used that contain gRNA target sites flanking the template. When the CRISPR nuclease and the appropriate gRNA(s) are present, the template is liberated from the vector. To avoid plasmid cloning, it is possible to use PCR-generated long dsDNA templates. Moreover, Quadros et al. (Genome Biol. 2017 May 17;18(1):92) developed Easi-CRISPR, a technique that allows making large mutations and to take advantage of the benefits of ssODNs. To create ssODNs longer than 200 bases, RNA encoding the repair template are *in vitro* transcribed and then reverse transcriptase is used to create the complementary ssDNA. Easi-CRISPR works well in mouse knock-in models, increasing editing efficiency from 1-10% with dsDNA to 25-50% with ssODNs. Although HDR efficiency varies across loci and experimental systems, ssODN templates generally provide the highest frequency of HDR edits.

The target site is not particularly limited and refers to a genomic site of interest that is present in the genome of cells to be genome edited. The target site in the genome designates mitochondrial DNA or genomic DNA and preferably genomic DNA. The target site is preferably but not necessarily a gene of interest (or target gene). The target site may also be, for example, a gene regulatory element, such as a promoter region or a cis-regulatory element.

In accordance with step (A) of the method of the invention one or more nucleic acid molecules encoding in expressible form the CRISPR nuclease as well as the guide RNA, wherein the CRISPR nuclease and/or the guide RNA are encoded by said one or more nucleic acid molecules in a form wherein the expression can be induced or repressed. In addition, the HDR template either directly (in the form a single-stranded or double-stranded DNA) or in expressible form encoded by a nucleic acid molecule (preferably an expression vector) are introduced into the cells within the cell population.

In the cases where the HDR template is in expressible form encoded by a nucleic acid molecule said nucleic acid molecule may be a separate nucleic acid molecule or the same nucleic acid molecule that encodes the CRISPR nuclease and/or the guide RNA. In the cases where the HDR template is directly introduced into the cell the HDR template may be a linear double-stranded or single-stranded DNA molecule. The linear double-stranded DNA molecule is preferably a linearized PCR product and the linear single-stranded DNA molecule is preferably a ssODN as described herein above.

The nucleic acid molecules used in accordance with of the present invention may be inserted into several commercially available vectors. Single vectors containing both the CRISPR nuclease and the gRNAs and optionally the HDR template are commercially available, thereby acting as an all-in-one vector. The method of the invention can alternatively be implemented by using two or three vectors containing the CRISPR nuclease, gRNA and the HDR template. It is also possible to use gRNA and/or HDR template-only vectors and to use cells in which the CRISPR nuclease has been integrated into the genome. The use of an all-in-one vector that expresses the gRNA, the CRISPR nuclease and optionally the HDR template is preferred since only one vector is to be introduced into the cells. A vector which can express the CRISPR nuclease and up to seven gRNAs is, for example, described in Sakuma et al, Sci Rep. 2014; 4: 5400.

Many single gRNA empty vectors (with and without the CRISPR nuclease) are available in the art. Likewise several empty multiplex gRNA vectors are available that can be used to express multiple gRNAs from a single plasmid (with or without the expression of the CRISPR nuclease). Finally, also vectors are available that only express the CRISPR nuclease (see https://www.addgene.org/crispr/empty-grna-vectors/).

Vector modification techniques are known in the art and, for example, described in Sambrook and Russel, 2001. Generally, vectors can contain one or more origins of replication (ori) and inheritance systems for cloning or expression, one or more markers for selection in the host, e.g., antibiotic resistance, and one or more expression cassettes. Suitable origins of replication include, for example, the Col E1, the SV40 viral and the M 13 origins of replication. The nucleic acid sequences inserted in the vector can e.g. be synthesized by standard methods, or isolated from natural sources. Ligation of the coding sequences to transcriptional regulatory elements and/or to other amino acid encoding sequences can be carried out using established methods. Such regulatory sequences are well known to those skilled in the art and include, without being limiting, regulatory sequences ensuring the initiation of transcription, initiation of translation, internal ribosomal entry sites (IRES) or 2A linkers (Owens, Proc. Natl. Acad. Sci. USA 98 (2001), 1471-1476) and optionally regulatory elements ensuring termination of transcription and stabilization of the transcript. Non-limiting examples for regulatory elements (e.g. ensuring the initiation of transcription) comprise a translation initiation codon, enhancers such as e.g. the SV40-enhancer, insulators and/or promoters, such as for example the cytomegalovirus (CMV) promoter, elongation factor-1 alpha (EF1-alpha), promoter, SV40-promoter, RSV-promoter (Rous sarcoma virus), the lacZ promoter, chicken beta-actin promoter, CAG-promoter (a combination of chicken beta-actin promoter and cytomegalovirus immediate-early enhancer), the gai10 promoter, human elongation factor 1a-promoter, AOX1 promoter, GAL1 promoter, CaM-kinase promoter, the lac, trp or tac promoter, the lacUV5 promoter, the *Autographa californica* multiple nuclear polyhedrosis virus (AcMNPV) polyhedral promoter or a globin intron in mammalian and other animal cells. Non-limiting examples for regulatory elements ensuring transcription termination include the V40-poly-A site, the tk-poly-A site, lacZ or AcMNPV polyhedral polyadenylation signals, which are to be included downstream of the nucleic acid sequence of the invention. Additional regulatory elements may include translational enhancers, Kozak sequences and intervening sequences flanked by donor and acceptor sites for RNA splicing, nucleotide sequences encoding secretion signals or, depending on the expression system used, signal sequences capable of directing the expressed polypeptide to a cellular compartment. Moreover, elements such as origin of replication, drug resistance gene, regulators (as part of an inducible promoter) may also be included.

Means and methods for the introduction for the nucleic acid molecule(s) expressing the CRISPR nuclease and/or the gRNAs and optionally (further) the HDR template into cells are known in the art and these methods encompass transducing or transfecting cells.

Transduction is the process by which foreign DNA is introduced into a cell by a virus or viral vector. Transduction is a common tool used by molecular biologists to stably introduce a foreign gene into a host cell's genome. Generally, a plasmid is constructed in which the genes to be transferred are flanked by viral sequences that are used by viral proteins to recognize and package the viral genome into viral particles. This plasmid is inserted (usually by transfection) into a producer cell together with other plasmids (DNA constructs) that carry the viral genes required for formation of infectious virions. In these producer cells, the viral proteins expressed by these packaging constructs bind the sequences on the DNA/RNA (depending on the type of viral vector) to be transferred and inserted into viral particles. For safety, none of the plasmids used contains all the sequences required for virus formation, so that simultaneous transfection of multiple plasmids is required to get infectious virions. Moreover, only the plasmid carrying the sequences to be transferred contains signals that allow the genetic materials to be packaged in virions, so that none of the genes encoding viral proteins are packaged. Viruses collected from these cells are then applied to the cells to be altered. The initial stages of these infections mimic infection with natural viruses and lead to expression of the genes transferred and (in the case of lentivirus/retrovirus vectors) insertion of the DNA to be transferred into the cellular genome. However, since the transferred genetic material does not encode any of the viral genes, these infections do not generate new viruses (the viruses are "replication-deficient"). In the present case transduction may be used to generate cells that comprise the CRISPR nuclease in their genome in expressible form.

Transfection is the process of deliberately introducing naked or purified nucleic acids or purified proteins or assembled ribonucleoprotein complexes into cells. Transfection is generally a non-viral based method.

Transfection may be a chemical-based transfection. Chemical-based transfection can be divided into several kinds: transfection using cyclodextrin, polymers, liposomes, calcium phosphate or nanoparticles. One of the cheapest methods uses calcium phosphate. HEPES-buffered saline solution (HeBS) containing phosphate ions are combined with a calcium chloride solution containing the DNA to be transfected. When the two are combined, a fine precipitate of the positively charged calcium and the negatively charged phosphate will form, binding the DNA to be transfected on its surface. The suspension of the precipitate is then added to the cells to be transfected (usually a cell culture grown in a monolayer). By a process not entirely understood, the cells take up some of the precipitate, and with it, the DNA. This process has been a preferred method of identifying many oncogenes. Other methods use highly branched organic compounds, so-called dendrimers, to bind the DNA and transfer it into the cell. Another method is the use of cationic polymers such as DEAE-dextran or polyethylenimine (PEI). The negatively charged DNA binds to the polycation and the complex is taken up by the cell via endocytosis. Lipofection (or liposome transfection) is a technique used to inject genetic material into a cell by means of liposomes, which are vesicles that can easily merge with the cell membrane since they are both made of a phospholipid bilayer. Lipofection generally uses a positively charged (cationic) lipid (cationic liposomes or mixtures) to form an aggregate with the negatively charged (anionic) genetic material. This transfection technology performs the same tasks in terms of transfer into cells as other biochemical procedures utilizing polymers, DEAE-dextran, calcium phosphate, and electroporation. The efficiency of lipofection can be improved by treating transfected cells with a mild heat shock. Fugene is a series of widely used proprietary non-liposomal transfection reagents capable of directly transfecting a wide variety of cells with high efficiency and low toxicity.

Transfection may also be a non-chemical method. Electroporation (gene electrotransfer) is a popular method, where transient increase in the permeability of cell membrane is achieved when the cells are exposed to short pulses of an intense electric field. Cell squeezing enables delivery of molecules into cells via cell membrane deformation. Sonoporation uses high-intensity ultrasound to induce pore formation in cell membranes. This pore formation is attributed mainly to the cavitation of gas bubbles interacting with nearby cell membranes since it is enhanced by the addition of ultrasound contrast agent, a source of cavitation nuclei. Optical transfection is a method where a tiny (~1 µm diameter) hole is transiently generated in the plasma membrane of a cell using a highly focused laser. Protoplast fusion is a technique in which transformed bacterial cells are treated with lysozyme in order to remove the cell wall. Following this, fusogenic agents (e.g., Sendai virus, PEG, electroporation) are used in order to fuse the protoplast carrying the gene of interest with the recipient target cell.

Finally, transfection may be a particle-based method. A direct approach to transfection is the gene gun, where the DNA is coupled to a nanoparticle of an inert solid (commonly gold), which is then "shot" (or particle bombardment) directly into the target cell's nucleus. Hence, the nucleic acid is delivered through membrane penetration at a high velocity, usually connected to microprojectiles. Magnetofection, or magnet-assisted transfection, is a transfection method that uses magnetic force to deliver DNA into target cells. Impalefection is carried out by impaling cells by elongated nanostructures and arrays of such nanostructures such as carbon nanofibers or silicon nanowires which have been functionalized with plasmid DNA.

The term "in expressible form" means that the one or more nucleic acid molecules may encode their constituents in a form that ensures that the guide RNAs and/or the HDR template (if being encoded) are transcribed and that the CRISPR nuclease (if being encoded) is transcribed and translated into the active enzyme in the cells.

In this connection the terms "expression can be induced" and "expression can be repressed"" also means that the one or more nucleic acid molecules may encode their constituents in a form that ensures that the guide RNAs (if being encoded) are transcribed and that the CRISPR nuclease (if being encoded) is transcribed and translated into the active enzyme in the cells, but in addition allows to control the timing of the expression of the guide RNAs and/or the CRISPR nuclease. The expression can be activated at desired point in the case of an inducible expression system and the repression of the expression can be stopped at a desired point in time in case a repressible expression system. Means and method for inducing or repressing expression will be discussed herein below.

According to step (B) of the first aspect of the invention the cells are cultured at least for a time wherein the cells double under growth conditions wherein the target locus within the cells of the cell population becomes modified by homology directed repair (HDR). The HDR mechanism can only be used by the cell when there is donor template with homology to the target locus present in the nucleus, mostly in G2 and S phase of the cell cycle. For this reason the donor template is introduced in step (A) so that the donor template is in the cell at the start of step (B). Culturing the cell under growth conditions at least for a time wherein the cells double ensures that the donor template reaches the nucleuses and that the cells reach the G2 and S phase of the cell cycle, wherein HDR mostly takes place. Hence, in step (B) the target locus within the cells that successfully underwent HDR is replaced by the donor template and consequently the target locus is no longer present in the genome of the cells.

According to (C) of the first aspect of the invention - that is only carried out after the completion of step (B) - either the expression of the CRISPR nuclease and/or the guide RNA is induced in the cells or the repression of the expression of the CRISPR nuclease and/or the guide RNA in steps (A) and (B) is stopped. In both cases only and for the first time during the method of the first aspect of the invention the CRISPR nuclease and/or the guide RNA comes together in the cells, so that the guide RNA can lead the CRISPR Cas nuclease to the target locus, whereby the CRISPR Cas nuclease becomes active.

As discussed, in the HDR-genome edited cells that are generated in step (B) the target locus is no longer present but replace by the donor template. It follows that in step (C) the CRPRS Cas nuclease becomes only active in those cells that were not HDR-genome edited in step (B).

As also discussed, the CRISPR nucleases to be used in accordance with the present invention are so-called BRAIN Engineered Cas proteins (BEC) nucleases. It can be taken from the below examples that the BEC nucleases display a novel mode of action that is to the best knowledge of the inventors not known from any prior art CRISPR nucleases. This novel mode of action enables and forms the basis of the various embodiments as described herein.

This novel mode of action can be described as "double-stranded DNA collateral cleavage activity". It is known from the prior art that certain CRISPR nucleases can display single-stranded DNA collateral cleavage activity (ssDNA; Cas 12) or single-stranded RNA collateral cleavage activity (ssRNA; Cas13); see Shashital (2018), Genome medicine; 10:32. These prior art CRISPR nucleases bind via a guide RNA to their target locus leading to collateral ssDNA or ssRNA collateral activity. However, as the genome of almost all organisms consists of dsDNA those prior art CRISPR nucleases are not able to target genomic DNA and are not suitable for genome editing.

The BEC nucleases used according to the invention display double-stranded DNA collateral cleavage activity. Hence, essentially all dsDNA within the cell the genome of which is to be edited can be cleaved by the BEC nucleases once they become activated by binding to their target locus. To the best knowledge of the inventors the present disclosure is the first report of CRISPR nucleases with double-stranded DNA collateral cleavage activity.

This novel activity of BEC nucleases ensures that in step (C) within said cell population the HDR-genome edited cells become enriched. The cells that were HDR-genome edited in step (B) are protected from the collateral activity of the BEC nuclease while the cells that were not HDR-genome edited in step (B) are removed from the cells population by the double-stranded DNA collateral cleavage activity of the active BEC nuclease. The dsDNA breaks in genomic DNA provided in trans are expected to deplete undesired cells without an introduction of the HDR template, and because of the collateral activity the intrinsic NHEJ repair mechanism of the cell is not able to prevent cell death. The unspecific dsDNA breaks in the genome kills the undesired cells.

While this in principle also works if the BEC nuclease, the gRNA and the donor template are added at the same time, the examples herein below show that efficiency of removing the undesired cells that were not HDR-genome edited and at the same time only obtaining the desired cells that were HDR-genome edited is increased if the HDR-genome editing is step (B) is carried out before the initiation of the double-stranded DNA collateral cleavage activity.

It is therefore preferred that the CRISPR nucleases to be used herein are not only structurally defined by displaying the required sequence homology to the exact sequences of the BEC nucleases but in addition functionally defined as displaying double-stranded DNA collateral cleavage activity and/or as being capable of depleting cells that repaired the dsDNA break as introduced by the active BEC nucleases at the target locus by NHEJ.

In accordance with a preferred embodiment of the first aspect of the invention in the form wherein the expression can be induced the expression of the CRISPR nuclease and/or the guide RNA is under the control of an inducible promoter, preferably a chemically inducible promoter.

Similarly, in accordance with a preferred embodiment of the first aspect of the invention in the form wherein the expression can be repressed the expression of the CRISPR nuclease and/or the guide RNA is under the control of a repressible promoter, preferably a chemically repressible promoter.

In the case of inducible expression, the guide RNA and/or the CRISPR nuclease is under the control of a promoter that is inactive and only becomes active in case a transactivator is present. The transactivator is preferably a chemical compound (such as alcohol a steroid) or can also be another external factor, such as temperature or light. On the other hand, in the case of repressible expression the guide RNA and/or the CRISPR nuclease is under the control of a promoter that is inactive and only becomes active in case a transrepressor is absent. The nature of the transrepressor can be the same as the nature of the transactivator, just the promoter works the other way round.

The inducible expression system is preferably a tet-on system and the repressible expression system is most preferably a tet-off system. In these systems gene expression is regulated by the presence or absence of tetracycline or one of its derivatives such as doxycycline. For instance, in a tet-on system tetracycline binds the rtTA transcription factor and allows it to bind DNA at the promoter. Gene expression is induced in the presence of tetracycline. In a Tet-Off system: tetracycline prevents the tTA transcription factor from binding DNA at the promoter. Gene expression is inhibited in the presence of tetracycline.

Also, the inducible expression system is preferably a L-rhamnose inducible expression system. The L-rhamnose-inducible promoter is capable of high-level recombinant protein expression in the presence of L-rhamnose, it is also tightly regulated in the absence of L-rhamnose by the addition of D-glucose. L-rhamnose is taken up by the RhaT transport system, converted to L-rhamnulose by an isomerase RhaA and then phosphorylated by a kinase RhaB. Subsequently, the resulting rhamnulose-1-phosphate is hydrolyzed by an aldolase RhaD into dihydroxyacetone phosphate, which is metabolized in glycolysis, and L-lactaldehyde. The latter can be oxidized into lactate under aerobic conditions and be reduced into L-1,2-propanediol under unaerobic conditions. The genes rhaBAD are organized in one operon which is controlled by the rhaPBAD promoter. This promoter is regulated by two activators, RhaS and RhaR, and the corresponding genes belong to one transcription unit which is located in opposite direction of rhaBAD. If L-rhamnose is available, RhaR binds to the rhaPRS promoter and activates the production of RhaR and RhaS. RhaS together with L-rhamnose in turn binds to the rhaPBAD and the rhaPT promoter and activates the transcription of the structural genes. However, for the application of the rhamnose expression system it is not necessary to express the regulatory proteins in larger quantities, because the amounts expressed from the chromosome are sufficient to activate transcription even on multi-copy plasmids. Therefore, only the rhaPBAD promoter has to be cloned upstream of the gene that is to be expressed. Full induction of rhaBAD transcription also requires binding of the CRP-cAMP complex, which is a key regulator of catabolite repression.

While this system preferably uses the *P*. *pastoris* LRA4 promoter L-rhamnose-inducible *P*. *pastoris* LRA4 promoter an L-rhamnose-inducible expression vector is also available, for example, from E. coli (Wegerer at al. (2008), BMC Biotechnol. 2008; 8: 2). The rhamnose-inducible rhaBAD promoter of *E. coli* can also be used in cells other then *E. coli* cells (Kelly et al. (2018), ACS Synth. Biol., 7(4):1056-1066).

The present invention relates in a second aspect to a method for the genome editing of cells at a target locus by homology directed repair (HDR) within a cell population and concurrently enriching within said cell population the HDR-genome edited cells, wherein the method comprises (A) introducing into the cells within the cell population a nucleic acid molecule, said nucleic acid molecule encoding in expressible form (i) a guide RNA comprising (a) a first segment comprising a nucleotide sequence that is complementary to a sequence at the target locus and (b) a second segment that interacts with the CRISPR nuclease of (C), and a nucleic acid molecule comprising, consisting of, or encoding (ii) a donor template with homology to the target locus; (B) culturing the cells at least for a time wherein the cells double under growth conditions wherein the target locus within the cells of the cell population becomes modified by homology directed repair (HDR), and (C) introducing after (B) into the cells within the cell population a CRISPR nuclease comprising or consisting of (a) an amino acid sequence of any one of SEQ ID NO: 1, 2 or 3; (b) an amino acid sequence being encoded by the nucleotide sequence of SEQ ID NO: 4, 5, 6 or 7; (c) an amino acid sequence being at least 80% identical to the amino acid sequence of (a), or (d) an amino acid sequence being encoded by a nucleic acid sequence being at least 80 % identical to the nucleotide sequence of (b), thereby enriching within said cell population the HDR-genome edited cells.

It has been discussed herein above in connection with the first aspect of the invention that it was found that the efficiency of removing the undesired cells that were not HDR-genome edited and at the same time only obtaining the desired cells that were HDR-genome edited is increased if the HDR-genome editing is step (B) is carried out before the initiation of the double-stranded DNA collateral cleavage activity in step (C).

This is achieved by the first aspect by the control of the expression of the gRNA and/or the CRISPR nuclease, so that only after step (B) both are expressly for the first time in step (C), wherein the gRNA, the CRISPR nuclease and the target locus together lead to the activity of the CRISPR nuclease.

The essentially same can be achieved by alternative means that are the subject-matter of the second to sixth aspect of the present invention of the invention. For this reason the definitions and preferred embodiment of the first aspect of the invention as describe herein above apply *mutatis mutandis* to the second to sixth aspect of the present invention as far as amenable for combination with these further aspects.

In accordance with the second aspect the nucleic acid molecule encoding in expressible form the guide RNA and the nucleic acid molecule comprising, consisting of, or encoding the donor template are introduced in step (A). The HDR event takes place in step (B). Only after step (B) the CRISPR nuclease is added to the cells in step (C) directly in the form a protein (and not encoded in expressible from by a nucleic acid molecule).

Also by this measure the gRNA, the CRISPR nuclease and the target locus may only be found together in a cell in step (C) and only the CRISPR nuclease becomes active, including its double-stranded DNA collateral cleavage activity.

Means for introducing proteins (or peptides) into living cells are known in the art and comprise but are not limited to microinjection, electroporation, lipofection (using liposomes), nanoparticle-based delivery, and protein transduction. Any one of these methods may be used in connection with step (a'). In this regard, the CRISPR nuclease to be introduced may either be isolated from their natural environment or recombinantly produced.

A liposome used for lipofection is a small vesicle, composed of the same material as a cell membrane (i.e., normally a lipid bilayer e.g. made of phospholipids), which can be filled with one or more protein(s) (e.g. Torchilin VP. (2006), Adv Drug Deliv Rev., 58(14):1532-55). To deliver a protein into a cell, the lipid bilayer of the liposome can fuse with the lipid bilayer of the cell membrane, thereby delivering the contained protein into the cell. It is preferred that the liposomes used in accordance with invention are composed of cationic lipids. The cationic liposome strategy has been applied successfully to protein delivery (Zelphati et al. (2001). J. Biol. Chem. 276, 35103-35110). As known in the art, the exact composition and/or mixture of cationic lipids used can be altered, depending upon the protein(s) of interest and the cell type used (Feigner et al. (1994). J. Biol. Chem. 269, 2550-2561). Nanoparticle-based delivery of Cas9 ribonucleoprotein and donor DNA for the induction of homology-directed DNA repair is, for example, described in Lee et al. (2017), Nature Biomedical Engineering, 1:889-90.

Protein transduction specifies the internalisation of proteins into the cell from the external environment (Ford et al (2001), Gene Therapy, 8:1-4). This method relies on the inherent property of a small number of proteins and peptides (preferably 10 to 16 amino acids long) being able to penetrate the cell membrane. The transducing property of these molecules can be conferred upon proteins which are expressed as fusions with them and thus offer, for example, an alternative to gene therapy for the delivery of therapeutic proteins into target cells. Commonly used proteins or peptides being able to penetrate the cell membrane are, for example; the antennapedia peptide, the herpes simplex virus VP22 protein, HIV TAT protein transduction domain, peptides derived from neurotransmitters or hormones, or a 9xArg-tag.

Microinjection and electroporation are well known in the art and the skilled person knows how to perform these methods. Microinjection refers to the process of using a glass micropipette to introduce substances at a microscopic or borderline macroscopic level into a single living cell. Electroporation is a significant increase in the electrical conductivity and permeability of the cell plasma membrane caused by an externally applied electrical field. By increasing permeability, protein (or peptides or nucleic acid sequences) can be introduced into the living cell.

The CRISPR nuclease may be introduced into the cells as an active enzyme or as a proenzyme. In the latter case the CRISPR nuclease is biochemically changed within the cells (for example by a hydrolysis reaction revealing the active site, or changing the configuration to reveal the active site), so that the proenzyme becomes an active enzyme.

The present invention relates in a third aspect to a method for the genome editing of cells at a target locus by homology directed repair (HDR) within a cell population and concurrently enriching within said cell population the HDR-genome edited cells, wherein the method comprises (A) introducing into the cells within the cell population a nucleic acid molecule, said nucleic acid molecule encoding in expressible form (i) a CRISPR nuclease comprising or consisting of (a) an amino acid sequence of any one of SEQ ID NO: 1, 2 or 3; (b) an amino acid sequence being encoded by the nucleotide sequence of SEQ ID NO: 4, 5, 6 or 7; (c) an amino acid sequence being at least 80% identical to the amino acid sequence of (a), or (d) an amino acid sequence being encoded by a nucleic acid sequence being at least 80 % identical to the nucleotide sequence of (b), and a nucleic acid molecule comprising, consisting of, or encoding (ii) a donor template with homology to the target locus; (B) culturing the cells at least for a time wherein the cells double under growth conditions wherein the target locus within the cells of the cell population becomes modified by homology directed repair (HDR), and (C) introducing after (B) into the cells within the cell population a guide RNA comprising (a) a first segment comprising a nucleotide sequence that is complementary to a sequence at the target locus and (b) a second segment that interacts with the CRISPR nuclease of A (i), thereby enriching within said cell population the HDR-genome edited cells.

In accordance with the third aspect the nucleic acid molecule encoding in expressible form the CRISPR nuclease and the nucleic acid molecule comprising, consisting of, or encoding the donor template are introduced in step (A). The HDR event takes place in step (B). Only after step (B) the gRNA is added to the cells in step (C) directly in the form an RNA molecule (and not encoded in expressible from by a nucleic acid molecule).

This measure again ensures that the gRNA, the CRISPR nuclease and the target locus may only be found together in a cell in step (C) and only then CRISPR nuclease becomes active, including its double-stranded DNA collateral cleavage activity.

Means for introducing a nucleic acid molecule such as a gRNA into cells have been discussed herein above, such a transfection or transduction.

The present invention relates in a fourth aspect to a method for the genome editing of cells at a target locus by homology directed repair (HDR) within a cell population and concurrently enriching within said cell population the HDR-genome edited cells, wherein the method comprises (A) introducing into the cells within the cell population a nucleic acid molecule comprising, consisting of, or encoding a nucleic acid molecule encoding in expressible form a donor template with homology to the target locus; and (B) culturing the cells at least for a time wherein the cells double under growth conditions wherein the target locus within the cells of the cell population becomes modified by homology directed repair (HDR), and (C) introducing after (B) into the cells within the cell population a ribonucleoprotein complex (RNP) comprising or consisting of a CRISPR nuclease comprising or consisting of (a) an amino acid sequence of any one of SEQ ID NO: 1, 2 or 3; (b) an amino acid sequence being encoded by the nucleotide sequence of SEQ ID NO: 4, 5, 6 or 7; (c) an amino acid sequence being at least 80% identical to the amino acid sequence of (a), or (d) an amino acid sequence being encoded by a nucleic acid sequence being at least 80 % identical to the nucleotide sequence of (b), in complex with a guide RNA comprising (a) a first segment comprising a nucleotide sequence that is complementary to a sequence at the target locus and (b) a second segment that interacts with the CRISPR nuclease; thereby enriching within said cell population the HDR-genome edited cells.

In accordance with the fourth aspect only the nucleic acid molecule comprising, consisting of, or encoding the donor template are introduced in step (A). The HDR event takes place in step (B). Only after step (B) the gRNA and the CRISPR nuclease are added in the form of a ribonucleoprotein complex (RNP) to the cells in step (C) directly in the form an RNA molecule (and not encoded in expressible from by a nucleic acid molecule).

Also this measure ensures that the gRNA, the CRISPR nuclease and the target locus may only be found together in a cell in step (C) and only then CRISPR nuclease becomes active, including its double-stranded DNA collateral cleavage activity.

RNPs are assembled *in vitro* and can be delivered to the cell by methods known in the art, for example, electroporation or lipofection. For introducing an RNP into a cell the same methods may be used as discussed for proteins herein above. RNPs are capable of cleaving the target site with comparable efficacy as nucleic acid-based (e.g. vector-based) CRISPR nucleases (Kim et al. (2014), Genome Research 24(6):1012-1019).

One aspect being related to the fourth aspect the invention relates to a method for the genome editing of cells at a target locus by homology directed repair (HDR) within a cell population and concurrently enriching within said cell population the HDR-genome edited cells, wherein the method comprises (A) introducing into the cells within the cell population a nucleic acid molecule comprising, consisting of, or encoding a nucleic acid molecule encoding in expressible form a donor template with homology to the target locus; and (B) culturing the cells at least for a time wherein the cells double under growth conditions wherein the target locus within the cells of the cell population becomes modified by homology directed repair (HDR), and (C) introducing after (B) into the cells within the cell population (i) a CRISPR nuclease comprising or consisting of (a) an amino acid sequence of any one of SEQ ID NO: 1, 2 or 3; (b) an amino acid sequence being encoded by the nucleotide sequence of SEQ ID NO: 4, 5, 6 or 7; (c) an amino acid sequence being at least 80% identical to the amino acid sequence of (a), or (d) an amino acid sequence being encoded by a nucleic acid sequence being at least 80 % identical to the nucleotide sequence of (b), or (ii) a nucleic acid molecule encoding the CRISPR nuclease of (a) in expressible form, and in addition a guide RNA comprising (a) a first segment comprising a nucleotide sequence that is complementary to a sequence at the target locus and (b) a second segment that interacts with the CRISPR nuclease or a nucleic caid in expressible; thereby enriching within said cell population the HDR-genome edited cells.

Also here the gRNA and the CRISPR nuclease are added to the cells in step (C), each independently either in the form of an RNA and protein, respectively of in the form of an expressible nucleic acid molecule encoding the gRNA and/or the CRISPR, respectively. In case both the gRNA and/or the CRISPR are encoded by an expressible nucleic acid molecule, this can be one nucleic acid molecule or two nucleic acid molecules.

The present invention relates in a fifth aspect to a method for the genome editing of cells at a target locus by homology directed repair (HDR) within a cell population and concurrently enriching within said cell population the HDR-genome edited cells, wherein the method comprises (A) introducing into the cells within the cell population a nucleic acid molecule encoding in expressible form (i) a guide RNA comprising (a) a first segment comprising a nucleotide sequence that is complementary to a sequence at the target locus and (b) a second segment that interacts with the CRISPR nuclease of (C), and a nucleic acid molecule comprising, consisting of, or encoding (ii) a donor template with homology to the target locus; and (B) culturing the cells at least for a time wherein the cells double under growth conditions wherein the target locus within the cells of the cell population becomes modified by homology directed repair (HDR), and (C) introducing after (B) into the cells within the cell population a nucleic acid molecule encoding in expressible form a CRISPR nuclease comprising or consisting of (a) an amino acid sequence of any one of SEQ ID NO: 1, 2 or 3; (b) an amino acid sequence being encoded by the nucleotide sequence of SEQ ID NO: 4, 5, 6 or 7; (c) an amino acid sequence being at least 80% identical to the amino acid sequence of (a), or (d) an amino acid sequence being encoded by a nucleic acid sequence being at least 80 % identical to the nucleotide sequence of (b), thereby enriching within said cell population the HDR-genome edited cells.

In accordance with the fifth aspect the nucleic acid molecule encoding in expressible form the guide RNA and the nucleic acid molecule comprising, consisting of, or encoding the donor template are introduced in step (A). The HDR event takes place in step (B). Only after step (B) the nucleic acid molecule encoding in expressible form the CRISPR nuclease is added to the cells in step (C). Hence, the CRISPR nuclease is added in the form a nucleic acid molecule, such a vector and not as in the second aspect in the in the form a protein.

This is a yet further measure ensuring that the gRNA, the CRISPR nuclease and the target locus may only be found together in a cell in step (C) and only the CRISPR nuclease becomes active, including its double-stranded DNA collateral cleavage activity.

The present invention relates in a sixth aspect to a method for the genome editing of cells at a target locus by homology directed repair (HDR) within a cell population and concurrently enriching within said cell population the HDR-genome edited cells, wherein the method comprises (A) introducing into the cells within the cell population a nucleic acid molecule encoding in expressible form (i) a CRISPR nuclease comprising or consisting of (a) an amino acid sequence of any one of SEQ ID NO: 1, 2 or 3; (b) an amino acid sequence being encoded by the nucleotide sequence of SEQ ID NO: 4, 5, 6 or 7; (c) an amino acid sequence being at least 80% identical to the amino acid sequence of (a), or (d) an amino acid sequence being encoded by a nucleic acid sequence being at least 80 % identical to the nucleotide sequence of (b), and a nucleic acid molecule comprising, consisting of, or encoding (ii) a donor template with homology to the target locus; and (B) culturing the cells at least for a time wherein the cells double under growth conditions wherein the target locus within the cells of the cell population becomes modified by homology directed repair (HDR), and (C) introducing after (B) into the cells within the cell population a nucleic acid molecule encoding in expressible form a guide RNA comprising (a) a first segment comprising a nucleotide sequence that is complementary to a sequence at the target locus and (b) a second segment that interacts with the CRISPR nuclease of (A)(i) thereby enriching within said cell population the HDR-genome edited cells.

In accordance with the sixth aspect the nucleic acid molecule encoding in expressible form the CRISPR nuclease and the nucleic acid molecule comprising, consisting of, or encoding the donor template are introduced in step (A). The HDR event takes place in step (B). Only after step (B) the nucleic acid molecule, for example, a vector encoding in expressible form the gRNA is added to the cells in step (C). Hence, the gRNA is added in the form a nucleic acid molecule, such a vector and not in the in the form of an RNA as in the third aspect of the invention.

Also here the gRNA, the CRISPR nuclease and the target locus can only be found together in a cell in step (C) and only then CRISPR nuclease becomes active, including its double-stranded DNA collateral cleavage activity.

According to a preferred embodiment of all the above aspects of the invention the method further comprises (D) isolating after (C) one or more cells, wherein the target locus has been genome edited by homology directed repair (HDR).

It is emphasized that not yet 100% of the cells may have been genome edited by HDR after step (C). Hence, in this case of the method of the above aspects of the invention step (D) may just be as easy as the collecting of one or more cells from a culture plate.

In general means and methods for an isolation step from a heterogeneous population of cells are known in the art. Non-limiting examples are single-cell dilution, laser capture microdissection, manual or automated cell picking, FACS and MACS.

In single-cell dilution a solution comprising cells is diluted in one or more steps until a solution with only a single cell is obtained. Laser capture microdissection is a method for isolating specific cells of interest from microscopic regions of tissue, cells or organisms. A laser is coupled into a microscope and focuses onto on a selected cell within a cell population. By movement of the laser by optics or the stage the focus follows a trajectory which is predefined by the user. This trajectory with the selected cell, also called element, is then cut out and separated from the adjacent cells. Manual cell picking is a simple, convenient, and efficient method for isolating single cells. Manual cell picking micromanipulators consist of an inverted microscope combined with micro-pipettes that are movable through motorized mechanical stages. Cell picking can also be implemented into an automated device. Fluorescence Activated Cell Sorting (FACS), a specialized type of flow cytometry with sorting capacity, is the most sophisticated and user-friendly technique for characterizing and defining different cell types in a heterogeneous cell population based on size, granularity, and fluorescence. FACS allows simultaneous quantitative and qualitative multi-parametric analyses of single cells. Magnetic-Activated Cell Sorting (MACS) is another commonly used passive separation technique to isolate different types of cells depending on their cluster of differentiation. It has been reported that MACS is capable of isolating specific cell populations with a purity >90%.

The invention also pertains to an isolated cell obtained by any of the above methods as well as a composition comprising this cell, wherein the composition is preferably an industrial composition, a diagnostic composition or a pharmaceutical composition. The pharmaceutical composition is preferred.

An industrial composition is intended to be used in industry, including agriculture. For instance, cells wherein a particular enzyme as the gene of interest has been introduced may be used in chemical production, biofuels, food & beverage, animal feeds, cosmetic products and consumer products.

A diagnostic composition is intended to be used in the diagnosis or a disease or condition. For instance, cells wherein a gene encoding a particular fluorescent protein of interest has been introduced, this protein may be used in diagnosis since it can be detected within an organism or a tissue sample.

The term "pharmaceutical composition" relates to a composition for administration to a patient, preferably a human patient. The pharmaceutical composition of the invention comprises the cells recited above. It may, optionally, comprise further molecules capable of altering the characteristics of the cells of the invention thereby, for example, stabilizing, modulating and/or activating their function. The composition is preferably in liquid form, e.g. (a) solution(s). The pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents etc. Compositions comprising such carriers can be formulated by well-known conventional methods. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1×10⁴ to 1×10⁸ cells per day. However, a more preferred dosage might be in the range of 1×10⁵ to 1×10⁷ cells and most preferably 5×10⁵ to 5×10⁶ cells per day.

The length of treatment needed to observe changes and the interval following treatment for responses to occur vary depending on the desired effect. The particular amounts may be determined by conventional tests which are well known to the person skilled in the art.

According to a preferred embodiment of all the above aspects of the invention the donor template (i) introduces (or inserts) one or more mutations at the target locus, (ii) inserts one or more nucleotides at the target locus, (iii) deletes one or more nucleotides at the target locus and/or (iv) substitutes one or more nucleotides at the target locus.

This can be achieved by designing the donor template accordingly. As discussed above, the donor template generally comprises homology arms that ensure that the donor template can replace the target locus by HDR. The sequence between the homology arms can comprise additional nucleotides or stretch(es) of nucleotide(s) as compared to the target site (insertion); can lack can one or more nucleotides or stretch(es) of nucleotide(s) as compared to the target site (deletion), and/or can comprise one or more different nucleotides or different stretch(es) of nucleotide(s) as compared to the target site (substitution). The stretch of nucleotides of an insertion can comprise or consist of one or more genes, optionally together with one or more expression regulatory sequences, such as a promoter.

Hence, HDR templates can be used to specifically modify a target locus, as needed, by the addition, deletion and/or substitution one or more nucleotides or stretch(es) of nucleotide(s).

According to a further preferred embodiment of all the above aspects of the invention the cells are cultured in step (B) at least for a time wherein the cells double 1.5 times, preferably 2 times, more preferably 2.5 times and most preferably 3.0 times.

The doubling time is the time it takes cells to double in number. Alternatively, doubling time is the time it takes for the cells to undergo at least one cell division. Means and methods for determining the doubling time for cells are known in the art.

The cell doubling time can as be determined, for example, on the basis of the cell culture monitoring data (Korzynaska and Zychwicz (2008), Biocybernetics and Biomedical Engineering, 28, Number(4):75-82).

According to a further preferred embodiment of all the above aspects of the invention the donor template comprises or consists of a nucleotide sequence with one or more intended mutations flanked by nucleotide sequences being homologous to the target locus.

The nucleotide sequences being homologous to the target locus are also referred to herein as homology arms. In the case of a double-stranded donor template the homology arms are preferably 30 to 800 bp, more preferably 300 to 500 bp. In the case of a single-stranded donor template the homology arms are preferably at least 300bp and more preferably at least 800 bp.

The sequence with one or more intended mutations between the arms can comprise the addition, deletion and/or substitution of one or more nucleotides or stretch(es) of nucleotide(s).

According to a different preferred embodiment of all the above aspects of the invention the cells within the population are capable of repairing double-stranded DNA breaks through non-homologous end joining (NHEJ).

Cells being capable of repairing double-stranded DNA breaks by NHEJ may repair the double-stranded DNA break as introduced by CRISPR nuclease by NHEJ. This is in particular undesired in case genome editing via a HDR template is desired. The BEC nucleases according to the present invention are capable of removing or preventing the occurrence of cells wherein the double-stranded DNA is repaired by NHEJ, in the presence of HDR template and of note also it its absence. It is believed that this is achieved by the above-described dsDNA collateral activity of the BEC nucleases according to the present invention.

In accordance with a further preferred embodiment of the first and second aspect of the invention, the cells within the population are prokaryotic cells. Alternatively and also preferred, they are eukaryotic cells, and are more preferably vertebrate cells, and most preferably mammalian cells.

According to a preferred embodiment of all the above aspects of the invention the cells within the population are prokaryotic cells or eukaryotic cells, preferably vertebrate cell, and most preferably mammalian cells.

The prokaryotic cells are preferably bacterial cells, such as *E. coli* or *B. subtilis* cells.

Eukaryotic cells are preferred as compared to prokaryotic cells. The eukaryotic cells can not only be chordate or vertebrate cells but can also be yeast cells, such as a *P*. *pastoris* or *A*. *niger* cells.

The vertebrate cells are preferably cells of a vertebrate (in particular mammalian such as human) cell line, organoids, primary cells, cells from a primary cell line, or pluripotent stem cells.

A mammalian cell line is a population of cells from a mammal which would normally not proliferate indefinitely but, due to mutation (that naturally occurred, e.g. in a tumor or by artificial mutagenesis), have evaded normal cellular senescence and instead can keep undergoing division. The cells can therefore be grown for prolonged periods *in vitro.*

An organoid is a miniaturized and simplified version of an organ produced *in vitro* in three dimensions that shows realistic micro-anatomy. They are derived from one or a few cells from a tissue, embryonic stem cells or induced pluripotent stem cells, which can self-organize in three-dimensional culture owing to their self-renewal and differentiation capacities.

Primary cells are cells taken directly from living tissue (e.g. biopsy material) and established for growth *in vitro.* These cells have undergone very few population doublings and are therefore more representative of the main functional component of the tissue from which they are derived in comparison to continuous (tumor or artificially immortalized) cell lines, thus generally representing a more representative model to the *in vivo* state. A primary cell line is a cell line that has been established from primary cells.

Pluripotent stem cells are cells that have the capacity to self-renew by dividing and to develop into the three primary germ cell layers of the early embryo and therefore into all cells of the adult body, but not extra-embryonic tissues such as the placenta. Embryonic stem cells and induced pluripotent stem cells are pluripotent stem cells. Embryonic stem cells are derived from the inner cell mass of a blastocyst, an early-stage pre-implantation embryo. Human embryos reach the blastocyst stage 4-5 days post fertilization, at which time they consist of 50-150 cells. They are preferably isolated from the embryo without the destruction of the embryo. Induced pluripotent stem cells (also known as iPS cells or iPSCs) are a type of pluripotent stem cell that can be generated directly from adult cells. The iPSC technology was pioneered by Shinya Yamanaka's lab in Kyoto, Japan, who showed in 2006 that the introduction of four specific genes encoding transcription factors could convert adult cells into pluripotent stem cells. The generation of iPSCs using Oct3/4 and/or a factor belonging to the Myc, Klf and Sox families of factors is described in WO 2009/144008.

According to another preferred embodiment of all the above aspects of the invention the method is an *in vitro* or *ex vivo* method.

An ex vivo method is a method being carried out of the context of a living organism. Similarly, *in vitro* methods are performed with microorganisms, cells, or biological molecules outside their normal biological context.

Both, *in vitro* and *ex vivo* methods exclude methods for treatment of the human or animal body by surgery or therapy and diagnostic methods practised on the human or animal body.

According to a yet further another preferred embodiment of all the above aspects of the invention the cells are synchronized after (A) and before (B).

The step of synchronizing and capturing cells at the S and G2 phases in step (B) can increase the efficiency of HDR-mediated genome editing; see, for example, Lin et al. (2014), eLife; 3: e04766. The synchronization and capturing of the cells at the S phase is preferred.

Cell cycle synchronization techniques are well-established and are reviewed, for example, in Jackman and O'Connor (2011), Curr Protoc Cell Biol; Chapter 8:Unit 8.3.

The present invention relates in a seventh aspect to one or more vector(s) comprising in expressible form (i) a CRISPR nuclease comprising or consisting of (a) an amino acid sequence of any one of SEQ ID NO: 1, 2 or 3; (b) an amino acid sequence being encoded by the nucleotide sequence of SEQ ID NO: 4, 5, 6 or 7; (c) an amino acid sequence being at least 80% identical to the amino acid sequence of (a), or (d) an amino acid sequence being encoded by a nucleic acid sequence being at least 80 % identical to the nucleotide sequence of (b), (ii) a guide RNA comprising (a) a first segment comprising a nucleotide sequence that is complementary to a sequence at the target locus; and (a) a second segment that interacts with the CRISPR nuclease of (i), wherein the CRISPR nuclease and/or the guide RNA are encoded by said one or more nucleic acid molecules in a form wherein the expression can be induced or repressed, and (iii) optionally a donor template with homology to the target locus.

The one or more vector(s) is preferably two vectors. The one vector preferably comprises the CRISPR nuclease and the guide RNA.

According to another preferred embodiment of all the above aspects of the invention the sequence identity of at least 80% is at least 85%, preferably at least 90% and most preferably at least 95%.

Amino acid sequence as well as nucleotide sequence analysis and alignments in connection with the present invention are preferably carried out using the NCBI BLAST algorithm (Stephen F. Altschul, Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402). The skilled person is aware of additional suitable programs to align nucleic acid sequences.

Also in connection with this preferred embodiment it is preferred that the CRISPR nucleases to be used herein are not only structurally defined but are in addition functionally defined as displaying double-stranded DNA collateral cleavage activity. Alternatively or additionally the CRISPR nucleases may be defined (i) as being capable of depleting cells that repaired the dsDNA break a introduced by the active BEC nucleases at the target locus by NHEJ (in cases where a HDR template is used), or (ii) as being capable of depleting cells comprising a target locus (in cases where no HDR template is used).

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show.
**Figure 1** - Schematic figure visualizing the strategy to co-transfect two vectors into *E. coli* to compare the DNA targeting mechanism of the BEC10 nuclease in comparison to FnCpf1.
**Figure 2** - Exemplary culture plates showing *E. coli* colonies 48h after transformation to visualize the different molecular mechanism of BEC10 in comparison to FnCpf1.
**Figure 3** - Exemplary culture plates showing *P. pastoris* colonies 48h after transformation to visualize the different genome editing mechanism of BEC10 in comparison to SpCas9.
**Figure 4** - Exemplary culture plates showing P. pastoris colonies 4 days after transformation to visualize the effect of a delayed BEC expression

The examples illustrate the invention.

### Example 1: Construction of a functional genome editing system for E. coli (BEC10 and FnCpf1) and Pichia pastoris (BEC10 and SpCas9)

### 1.1 CRISPR/BEC-Ec and FnCpf1-Ec vector systems for genome editing in E. coli BW25113

The necessary genetic elements for inducible expression of the BEC10 or FnCpf1 nucleases and for the constitutive expression of the guide RNA (gRNA) transcription were provided on three separate vectors (CRISPR/BEC10-Ec, CRISPR/FnCpf1-Ec and CRISPR/gRNA-Ec)

In the following, the construction of the CRISPR/BEC10-Ec vector and the CRISPR/gRNA-Ec systems are described. The CRISPR/FnCpf1-Ec vector systems was constructed in an analogous approach as the CRISPR/BEC10-Ec vector

### Design of the BEC10 Coli protein expression vector

The synthetic 3696 bps BEC10 nucleotide sequence was codon optimized for expression in *E. coli BW25113,* using a bioinformatics application provided by the gene synthesis provider GeneArt (Thermo Fisher Scientific, Regensburg, Germany), SEQ ID NO: 7. For protein expression, the resulting synthetic gene was fused to the inducible araC-ParaBAD inducible promoter system (SEQ ID NO: 8) and the fdT terminator (SEQ ID NO: 9) (Otsuka & Kunisawa, Journal of Theoretical Biology 97 (1982), 415-436) The final BEC10_*E*. *coli* protein expression cassette was inserted by Gibson Assembly Cloning (NEB, Frankurt, Germany) into an *E. coli* shuttle vector, containing all necessary genetic elements for episomal propagation and selection of recombinant *E. coli* cells.

### CRISPR/BEC10-Ec vector system

The complete nucleotide sequence of the constructed CRISPR/BEC10-Ec vector system is provided as SEQ ID NO: 10.

### CRISPR/FnCpf1-Ec vector system

The complete nucleotide sequence of the constructed CRISPR/fnCpf1-Ec vector system is provided as SEQ ID NO: 11.

### Design of the guide RNA (gRNA) expression vector

The expression of the chimeric gRNA for specific kanamycin gene targeting by BEC10 or FnCpf1 DNA nucleases was driven by the SacB RNA polymerase II promoter from *Bacillus megaterium* (SEQ ID NO: 12) (Richhardt et al., Applied Microbiology Biotechnology 86 (2010), 1959-1965) and terminated using the transcription T1 and T2 termination region of the *E. coli* rrnB gene (SEQ ID NO: 13) (Orosz et al., European Journal of Biochemistry 201 (1991), 653-659). The chimeric gRNA was composed of a constant 19 bps BEC family Stem-Loop sequence (SEQ ID NO: 14 (which also works for the FnCpf1 nuclease) fused to the kanamycin target-specific 24 bps spacer sequence (SEQ ID NO: 15) located inside the kanamycin resistance gene of the CRISPR/BEC10-Ec or CRISPR/FnCpf1-Ec vector

The final gRNA expression cassette was inserted by Gibson Assembly Cloning (NEB, Frankurt, Germany) into an *E. coli* shuttle vector, containing all necessary genetic elements for episomal propagation and selection of recombinant *E. coli* cells.

The construction of the final CRISPR/gRNA-Ec vector system was mediated by Gibson Assembly Cloning (NEB, Frankfurt, Germany).

The identity of all cloned DNA elements was confirmed by Sanger-Sequencing at LGC Genomics (Berlin, Germany).

The complete nucleotide sequence of the constructed CRISPR/gRNA-Ec vector system is provided as SEQ ID NO: 16.

### 1.2 E. coli cultivation and transformation

### Transformation of competent E. coli BW25113 cells

In brief, a single colony of *E. coli BW25113* was inoculated in 5 ml LB-Kan medium and incubated for 12 to 14 h at 37°C on a horizontal shaker at 200rpm. Overnight grown pre-cultures were diluted into fresh 60 ml LB medium to an optical density at 600 nm (OD600) of 0.06. The inoculated medium was incubated at 30°C on a horizontal shaker at 200 rpm until the culture reached an optical density at OD600 of 0.2. 600 µl. 20 % arabinose was added and the cells were incubated at 30°C at 200rpm until the culture reached an optical density at OD600 of 0.5. Cells were transferred into a 50 ml conical tube and harvested by centrifugation at 4°C for 5 min and 4000 x g. Pelleted cells from 50 ml culture were resuspended in 60 ml water and centrifuged at 4°C for 5 min and 4000 x g.

A washing procedure was performed and the cells were resuspended in 30 ml 10 % glycerin following a centrifugation at 4°C for 5 min and 4000 x g. In a second washing step, the cells were resuspended in 6 ml 10 % glycerin following a centrifugation at 4°C for 5 min and 4000 x g. In the final step, the cells were resuspended in 150 µl 10 % glycerin. Aliquots of 25 µl competent cells were stored at - 80°C until use. For transformation procedure, aliquots of competent cells were thawed and 50 ng plasmid DNA was added. Prepared cells were electroporated using 1800 V, 25 µF, 200 Ohm for 5 msec. Subsequently, 975 µL of NEB^{®} 10-beta/Stable Outgrowth Medium was added and 100 µl of the suspension was plated on selective agar plates.

### 1.3 CRISPR/BEC10-Pp vector systems for genome editing in Pichia pastoris

The necessary genetic elements for constitutive expression of the BEC10 and the guide RNA (gRNA) transcription were provided in an all-in-one CRISPR/BEC10-Pp vector system.

### Design of the BEC10 protein expression cassette

The synthetic 3696 bps BEC10 nucleotide sequence was codon optimized for expression in yeast cells, using a bioinformatics application provided by the gene synthesis provider GeneArt (Thermo Fisher Scientific, Regensburg, Germany), SEQ ID NO: 6. Additionally, the DNA nuclease coding sequence was 5' extended by a sequence encoding a SV40 nuclear localization signal (NLS) SEQ ID NO: 17 (Kalderon et al., Cell 39 (1984), 499-509). For protein expression, the resulting synthetic 3723 bps gene was fused to the constitutive and bidirectional *P. pastoris* HTX1 promoter (SEQ ID NO: 18) (Weninger et al., Journal of Biotechnology 235 (2016), 139-149) and the P. *pastoris* AOX1TT terminator (SEQ ID NO: 19) (Weninger et al., Journal of Biotechnology 235 (2016), 139-149). The final BEC10 protein expression cassette was inserted by Gibson Assembly Cloning (NEB, Frankurt, Germany) into an *E. coli*/*P. pastoris* shuttle vector pPpT6e (EP 3572512 A1), containing all necessary genetic elements for episomal propagation and selection of recombinant *E. coli* and *P. pastoris* cells:
For vector propagation and selection of recombinant *E. coli* cells, the plasmid contained the pUC derived high-copy ColE1 origin of replication and the kanMX6 marker gene (SEQ ID NO: 20) under the control of the bifunctional ILV5/synthetic Em72 promoter (SEQ ID NO: 21), (Weninger et al., Journal of Biotechnology 235 (2016), 139-149), conferring kanamycin resistance to *P*. *pastoris and E. coli* respectively. The Pichia autonomously replicating sequence 1 (PARS1) (SEQ ID NO: 22) allowed episomal replication of the pPpT6e shuttle plasmid in *P. pastoris* cells.

### Design of the guide RNA (gRNA) expression cassette

Ribozyme-based technology was applied to liberate the chimeric gRNA from a RNA polymerase II transcript for specific Ade2 gene targeting by BEC10 DNA nuclease in P. *pastoris* (Gao & Zhao, Journal of Integrative Plant Biology 56 (2014), 343-349). The bidirectional HTX1 RNA polymerase II promoter was used for expression of 5' cleaving hammerhead (HH) and 3' cleaving hepatitis delta virus (HDV) ribozyme flanked gRNA (Weninger et al., Journal of Biotechnology 235 (2016), 139-149).

The chimeric gRNA was composed of a constant 19 bps BEC family Stem-Loop Sequence (SEQ ID NO: 14) fused to the Ade2 target-specific 24 bps spacer sequence (SEQ ID NO: 23). The target spacer sequence was identified in the *P*. *pastoris* Ade2 gene (SEQ ID NO: 24) downstream to the nuclease BEC10 specific PAM motive 5'-TTN-3'.

The complete RNA expression cassette composed of the HTX1 RNA polymerase II promoter and the designed HH/HDV ribozyme-flanked chimeric gRNA was provided as a synthetic gene fragment by GeneArt (Thermo Fisher Scientific, Regensburg, Germany).

The construction of the all-in-one CRISPR/BEC10-Pp vector system was completed by cloning the synthetic RNA expression cassette in the prepared *E. coli*/*P. pastoris* pPpT6e shuttle vector, containing the BEC10 DNA nuclease expression cassette. The construction of the final CRISPR/BEC10-Pp vector system was mediated by Gibson Assembly Cloning (NEB, Frankfurt, Germany).

The identity of all cloned DNA elements were confirmed by Sanger-Sequencing at LGC Genomics (Berlin, Germany).

### CRISPR/BEC10-Pp all-in-one-vector system

The complete nucleotide sequence of the constructed CRISPR/BEC10-Pp vector system is provided as SEQ ID NO: 25.

### 1.4 CRISPR/SpCas9-Pp vector system for genome editing in P. pastoris

The necessary genetic elements for constitutive expression of SpCas9 (*S. pyogenes* Cas9) DNA nuclease and for single guide RNA transcription were provided in an all-in-one CRISPR/SpCas9_Pp vector system.

### Design of the SpCas9 protein expression cassette

Based on the published SpCas9 nucleotide sequence from *Streptococcus pyogenes,* (Deltcheva et al., Nature 471 (2011), 602-607) DNA synthesis of the yeast codon optimized SpCas9 coding sequence was ordered at GeneArt (Thermo Fisher Scientific, Regensburg, Germany), (SEQ ID NO: 26) for expression in P. *pastoris.* For nuclear translocation, the SpCas9 DNA nuclease coding sequence was 5' extended by a sequence encoding a SV40 nuclear localization signal (NLS) (SEQ ID NO: 17). Following the described protein expression strategy for BEC10 DNA nuclease, the resulting synthetic 4134 bps SpCac9 gene was fused to the constitutive and bidirectional *P. pastoris* HTX1 promotor (SEQ ID NO: 18) and the P. *pastoris* AOX1TT terminator (SEQ ID NO: 19) (Weninger et al., Journal of Biotechnology 235 (2016), 139-149). The final SpCas9 protein expression cassette was inserted by Gibson Assembly Cloning (NEB, Frankurt, Germany) into an *E. coli*/*P. pastoris* pPpT6e shuttle vector, harboring the identical genetic elements for propagation and selection as already described for the CRISPR/BEC10-Pp vector system.

### Design of the guide RNA expression (gRNA) cassette

Ribozyme-based technology was applied to liberate the chimeric gRNA from from a RNA polymerase II transcript for specific Ade2 gene targeting by SpCas9 DNA nuclease in P. *pastoris* (Gao & Zhao, Journal of Integrative Plant Biology 56 (2014), 343-349). The bidirectional HTX1 RNA polymerase II promotor was used for expression of 5' cleaving hammerhead (HH) and 3' cleaving hepatitis delta virus (HDV) ribozyme flanked gRNA (Weninger et al., Journal of Biotechnology 235 (2016), 139-149).

The chimeric gRNA was composed of the Ade2 target-specific 20 bps spacer sequence (SEQ ID NO: 27) fused to the 80 bps SpCas9 specific sgRNA sequence (SEQ ID NO: 28) The target spacer sequence was identified in the *P*. *pastoris* Ade2 gene (SEQ ID NO: 24) downstream to the nuclease SpCa9 specific PAM motive 5'-NGG-3'.

The complete RNA expression cassette composed of the HTX1 RNA polymerase II promoter and the designed HH/HDV ribozyme-flanked chimeric gRNA was provided as a synthetic gene fragment by GeneArt (Thermo Fisher Scientific, Regensburg, Germany).

To generate the final CRISPR/SpCas9-Pp vector system, the synthetic RNA transcription cassette was cloned by Gibson Assembly Cloning (NEB, Frankfurt, Germany) into the prepared *E. coli*/*P. pastoris* pPpT6e shuttle vector, containing the SpCas9 DNA nuclease expression cassette. The identity of all cloned DNA elements were confirmed by Sanger-Sequencing at LGC Genomics (Berlin, Germany).

### CRISPR/SpCas9-Pp all-in-one-vector system

The complete nucleotide sequence of the constructed CRISPR/SpCas9-Pp vector system is provided as SEQ ID NO: 29.

### 1.5 Design of a homology directed repair template (HDR-template)

The 1998 bps Ade2 BEC10 and spCas9 HDR-template was designed to generate a site-specific deletion of 1692 bps in the chromosomal *P. pastoris* Ade2 gene by homologous recombination. Successful homologous recombination resulted in complete Ade2 gene deletion. Within the HDR-template, the introduced Ade2 gene deletion was flanked by 1022 bps and 976 bps sequences homologous to the chromosomal target region. The successful recombination event mediated by the designed HDR-template abolished the already described PAM and protospacer regions in the chromosomal Ade2 gene to prevent the programmed gRNA / BEC10 or gRNA / spCas9 DNA nuclease complex to target the *P. pastoris* genome again. Furthermore, the introduced gene deletion resulted in Ade2 mutant clones, which were easily recognized by the red color of the colonies, since the mutant cells, deprived of adenine, accumulate red purine precursors in their vacuoles (Ugolini et al., Curr Genet (2006), 485-92).

The complete sequence of the Ade2 HDR-template for BEC10 and SpCas9 is provided as SEQ ID NO: 30.

### 1.6 Pichia pastoris cultivation and transformation

### Preparation of competent P. pastoris CBS7435 cells

Preparation and transformation of competent *P*. *pastoris* CBS7435 cells were performed as described by Wu & Letchworth, Biotechniques (2014), 36, 152-154. In brief, a single colony of *P. pastoris* was inoculated in 5 ml YPD medium and incubated for 6 to 8 h at 30°C on a horizontal shaker at 250 rpm. Cells of the preculture were diluted into 100 ml YPD medium to an optical density at 600 nm (OD600nm) of 0.0025.

The inoculated medium was incubated overnight at 30°C on a horizontal shaker at 250 rpm until the culture reached an optical density at OD600nm of 1.0 to 2.0. Cells were harvested by centrifugation for 5 min and 4302 x g at room temperature (RT). The cell density of the culture (cells/mL) was estimated from the optical density at OD600nm according to the following relationship: 1 x OD600nm = 5 × 10⁷ cells/mL. For each transformation, 8 x 10⁸ cells were suspended in 8 mL of 100 mM LiAc, 10 mM DTT, 0.6 M sorbitol and 10 mM Tris-HCI. The resuspended cells were incubated for 30 min at RT.

The cells were then pelleted by centrifugation for 5 min and 3999 x g at RT and resuspended in 1.5 mL of ice-cold 1M sorbitol. The cells were transferred to a 1.5-mL microcentrifuge tube, washed three times with 1.5 mL ice-cold 1 M sorbitol and finally resuspended in 80 µl 1 M ice-cold sorbitol at a final cell density of about 10¹⁰ cells/mL.

### Transformation of competent P. pastoris cells

One aliquot of prepared competent cells was mixed with 1 µg of supercoiled plasmid DNA in 5 µL water. For repair of CRISPR/BEC10- or CRISPR/Cas9-induced DNA damages by homologous recombination, 1.5 µg of a linear double-stranded DNA fragment was added to the cells. The total volume of 5µl DNA solution was not exceeded. The DNA-cell mixture was transferred to a precooled 0.2-cm gap vial and incubated for 5 min on ice. The electroporating pulse was applied at 2 kV, 25 µF, 200 Ω using a Bio-Rad Gene Pulser Xcell electroporation system (Bio-Rad Laboratories, Munich, Germany). The electroporated cells were immediately diluted in 1 mL of ice-cold regeneration medium (0.5 x YPD, 0.5 M sorbitol) and transferred into a 2 ml microcentrifuge tube. After incubation for 1 h without agitation and 2 h on a horizontal shaker at 250 rpm, the transformed cells were plated on appropriate selective agar plates, depending on the experimental setup.

### Plating of transformed P. pastoris cells

For analyzing the efficiencies of various gRNA spacers, in the absence of any homologous repair template, transformed cells were plated on YPD agar plates containing 200 µg/mL geneticin (G418) and incubated at least for 2 days at 30°C.

Ade2 gene disruption and induced red phenotype of mutated P. pastoris cells by BEC10-gRNA- and Cas9-gRNA-mediated integration of co-transformed homologous repair template in the Ade2 target gene was visualized using a simple colony color filter assay: Transformed cells were plated on NC transfer membrane filters (Merck Chemicals, Darmstadt, Germany), applied directly to the surface of YPD agar plates supplemented with 200 µg/mL geneticin (G418) and 50 µg/mL adenine. After incubation for at least 2 days at 30 °C, the filters with grown cells were transferred onto minimal media agar plates supplemented with 200 µg/mL geneticin (G418) and 5 µg/mL adenine

### Example 2: Vector targeting in E. coli to demonstrate the novel DNA targeting mechanism of BEC family nucleases in comparison to FnCpf1

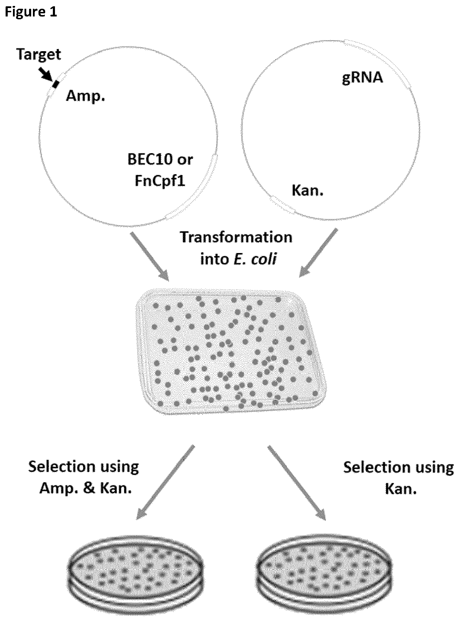

To demonstrate the novel DNA targeting mechanism of BEC family nucleases experiments were carried out using two different vectors co-transformed into *E. coli.* In two different approaches, the CRISPR/BEC10-Ec vector or the CRISPR/FnCpf1-Ec vector was co-transformed together with the CRISPR/gRNA-Ec vector including a spacer sequence targeting the Ampicillin resistance gene located on the CRISPR/BEC10-Ec vector or the CRISPR/FnCpf1-Ec vector (Schematic drawing of the experimental setup is shown in Fig. 1). Using a nuclease with a classical DNA targeting activity (double strand break) the activation of the CRISPR nuclease would linearize the CRISPR/BEC10-Ec or the CRISPR/FnCpf1-Ec vector leading to a disruption of the open reading frame of the Ampicillin resistance gene and prevent the propagation of the vector. Therefore, the cells would become sensitive for Ampicillin, due to deletion of the Ampicillin resistance gene located on the CRISPR/BEC10-Ec or the CRISPR/FnCpf1-Ec vector, but remain resistant against Kanamycin, since the Kanamycin resistance gene located on the CRISPR/gRNA-Ec vector remains intact.

To directly compare the DNA targeting mechanism of a BEC family (BEC10) and a classical (FnCpf1) nuclease the following experiments were carried out:
1. The CRISPR/BEC10-Ec vector and the CRISPR/gRNA-Ec vector were co-transformed into *E. coli* cells and grown/selected on plates containing Ampicillin & Kanamycin and plates containing only Kanamycin.
2. The CRISPR/FnCpf1-Ec vector and the CRISPR/gRNA-Ec vector were co-transformed into *E. coli* cells and grown/selected on plates containing Ampicillin & Kanamycin and plates containing only Kanamycin.
3. Negative control (NC) experiments for setup 1 and 2 were carried out using the same experimental approaches but with a nonsense spacer sequence (does not match a sequence in one of the vectors or the *E. coli* genome) in the CRISPR/gRNA-Ec vector.

All plates were visually evaluated by counting the number of grown colonies.

### Results

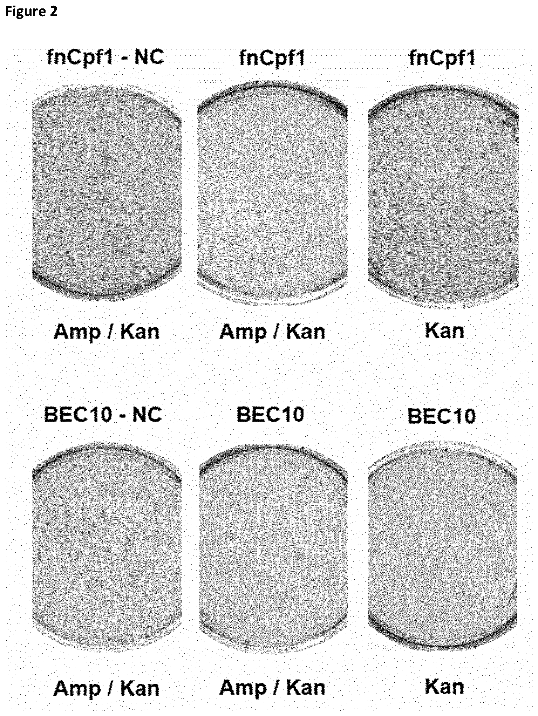

All experiments were carried out in 5 biological replicates and the results obtained from these replicates were combined to evaluate the DNA targeting mechanism of BEC10 in comparison to FnCpf1 (Exemplary plates are shown in Figure 2).

### FnCpf1

The results obtained using the FnCpf1 nuclease showed the expected outcome. The plates containing the Kanamycin antibiotic showed a comparable number of grown *E. coli* colonies to the negative control (NC) because the cells were still resistant to Kanamycin as the resistance gene is located on the CRISPR/gRNA-Ec vector that is not targeted by the gRNA. In contrast to this, the plates containing Ampicillin and Kanamycin showed a strong colony reduction (>99%) in contrast to the negative control because the vector containing the Ampicillin resistance gene (CRISPR/FnCpf1-Ec) was targeted and depleted by the FnCpf1 nuclease.

### BEC10

Surprisingly, the results obtained using the BEC10 nuclease showed a different growth pattern. The plates containing the Kanamycin antibiotic and the plates containing the Ampicillin and Kanamycin antibiotic showed a strong colony reduction (>99%) in comparison to the negative control. As described for the FnCpf1 experiments, the colony reduction was expected on plates containing Ampicillin and Kanamycin (targeting/depletion of the Ampicillin containing vector). In addition to this, the plates containing only Kanamycin showed no colony reduction using the FnCpf1 nuclease but a strong colony reduction using the BEC10 nuclease.

### Conclusion

The explanation for these significant differences between the BEC10 and FnCpf1 results is the novel mode of action of the BEC family nucleases: The initial sequence specific binding of the spacer sequence (incorporated into the gRNA) to the protospacer region (target region) on the vector activates the BEC nuclease and triggers an unspecific targeting of double stranded DNA. Therefore, the DNA targeting activity of the BEC nucleases (once specifically activated) degrades dsDNA and is able to "jump" to other DNA strands inside of the same cell. This is a type of reaction that can be described as collateral activity, since even DNA without a target region is degraded once the nuclease is activated.

In the shown experiment, the BEC nuclease was activated by the protospacer sequence located on the CRISPR/BEC10-Ec vector and once activated the activity "jumped" over and targeted the CRISPR/gRNA-Ec and/or the *E. coli* genome leading to the strong colony reduction on plates containing Kanamycin only.

To our knowledge, a nuclease that is specifically activated by a spacer/protospacer pairing and once activated has collateral dsDNA cutting activity has never been described before. This novel activity pattern can be used in various applications where classical CRISPR nucleases are non-functional or have a limited applicability (e.g. Example 3).

### Example 3:

### Precise genome editing in P. pastoris using the BEC10 nuclease in comparison to SpCas9

To demonstrate one of the advantages of the novel mode of action of BEC family nucleases, genome editing experiments were carried out in an organism that is natively capable to perform non-homologous end joining (NHEJ) DNA repair.

### Experimental Setup:

In this example, the CRISPR/BEC10-Pp or CRISPR/SpCas9-Pp vector system and HDR template were used to knock out the Ade2 gene in *P. pastoris.*

Ade2 is a non-essential gene of *P*. *pastoris* but a knockout leads to a red phenotype of the colonies, since the mutant cells accumulate red purine precursors in their vacuoles (Ugolini et al., Curr Genet (2006), 485-92) . Due to this easy readout, the knockout of the Ade2 gene can be utilized as a screening system to monitor the ability of CRISPR Cas proteins to function as a genome-editing tool.

In this approach, the knockout of the Ade2 gene was used to monitor the genome editing activity of the BEC10 nuclease in comparison to SpCas9 in two experimental setups.
1. Ade2 knockout via NHEJ (without using a HDR template)
2. Specific Ade2 knockout using homology directed repair (introduction of a HDR template leading to a site-specific deletion of the Ade2 gene also eliminating the PAM and protospacer sequence)

In summary, the CRISPR/BEC10-Pp or CRISPR/SpCas9-Pp expression constructs with or without a homology directed repair template were transformed into *P*. *pastoris* cells and plated as described in Example 1.6.

In parallel, negative control experiments using the CRISPR/BEC10-Pp or CRISPR/SpCas9-Pp expression constructs lacking a spacer sequence targeting the Ade2 gene were performed to demonstrate the dependency of the Cas proteins to be guided to the target DNA region by a specific spacer.

After transformation and 48 h incubation at 30°C the culture plates were analyzed by counting the number of grown colonies and by the evaluation of their phenotype (red or white).

### Results:

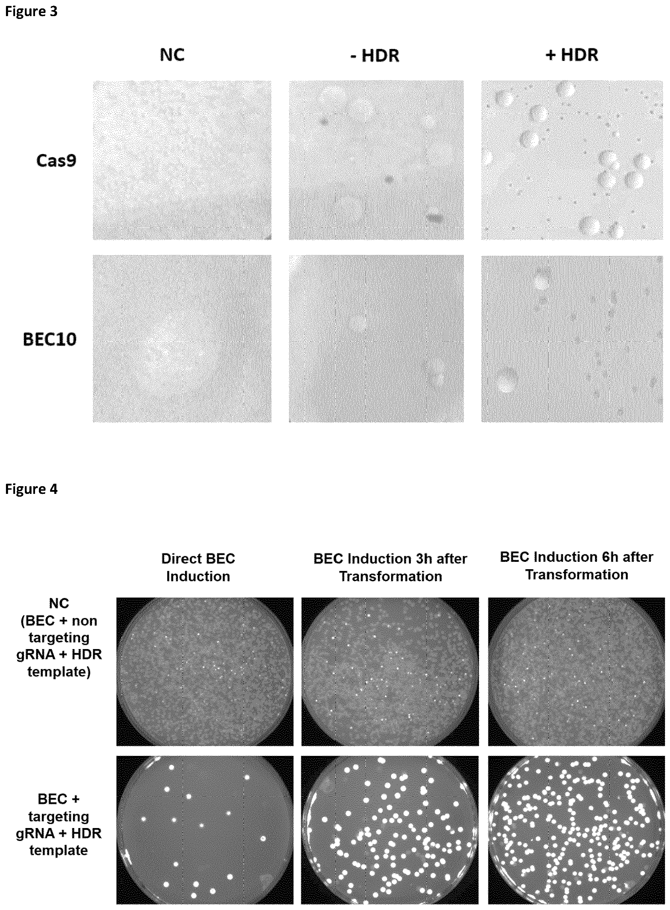

All experiments were carried out in 5 biological replicates and the results obtained from these replicates were combined to evaluate the genome editing activity of BEC10 in comparison to SpCas9 (Exemplary plates are shown in Figure 3).

In the first experimental setup the CRISPR/BEC10-Pp or CRISPR/SpCas9-Pp expression construct was transformed into *P. pastoris* cells without using a HDR template (-HDR). In comparison to the negative control experiments the active SpCas9 and BEC10 nuclease lead to a strong colony reduction (>99%) with an even stronger decrease of the colonies when using the BEC10 nuclease. Furthermore, in the experimental approach using the Cas9 nuclease ≈ 40% of the remaining cells showed a red (Ade2 knockout) phenotype in contrast to the cells treated with the BEC10 nuclease where 0% of the cells showed a red phenotype.

In the second experimental setup the CRISPR/BEC10-Pp or CRISPR/SpCas9-Pp expression construct was transformed into *P. pastoris* cells together with the HDR template (+HDR). In comparison to the negative control experiments the active SpCas9 and BEC10 nucleases lead to a strong colony reduction (>98%) which was a slightly less dramatic decrease in the overall cell numbers compared to the experiments without the HDR template. Furthermore, the majority of surviving cells showed an Ade2 knockout phenotype for SpCas9 and BEC10 (SpCas9 ≈ 73% / BEC10 ≈ 90%). Additionally, 40 colonies (20 SpCas9 treated and 20 BEC10 treated) showing an Ade2 knockout phenotype were further analyzed by Sanger sequencing showing that 14 out of the 20 colonies treated with Cas9 had incorporated the HDR template into their genome and 6 were edited by NHEJ. In contrast to this, 20 out of 20 colonies analyzed after the BEC10 treatment showed the introduction of the HDR template into the Ade2 gene into their genome.

### Conclusion:

Even though the SpCas9 and BEC nucleases both lead to a significant overall colony reduction after activation, the Ade2 gene editing showed unequivocally different results between both nucleases.

As *P. pastoris* is an organism that is intrinsically able to repair DNA double strand breaks using NHEJ, the targeting of the Ade2 gene using SpCas9 leads to Ade2 knockout colonies due to frameshift mutations caused by the faulty NHEJ mechanism. In contrast to this, the cells treated with the BEC10 nuclease did not show Ade2 knockout colonies, which can be explained by the novel mode of action of the BEC family nucleases. BEC family nucleases exhibit a completely different mode of action which prevents NHEJ because the activation of the BEC nuclease induces a collateral dsDNA activity leading to cell death without giving the cell the opportunity to prevent death by NHEJ derived DNA repair.

In contrast to this, Ade2 knockout colonies were present for SpCas9 and BEC10 when using a HDR template to knockout the gene. For SpCas9 the introduced dsDNA break inside the Ade2 gene forces the cell to repair this break. To do so, the cell has two options: A: introduce the HDR template or B: repair of the DNA break using NHEJ. As *P. pastoris* prefers homology directed repair over NHEJ ≈ 70% of the edited cells introduced the HDR template into their genome and ≈ 30% of the edited cells performed NHEJ mediated DNA repair.

In contrast to this, the BEC10 nuclease prevents the cells from NHEJ mediated DNA repair by killing the cells, once the BEC nuclease is activated. Therefore, the BEC10 nuclease forces the cells to introduce the HDR template into their genome before the BEC nuclease is activated. As the PAM and protospacer sequence within the genome are deleted by the integration of the HDR template, the protospacer sequence matching the spacer sequence of the BEC gRNA is no longer present in the genome and the BEC nuclease stays inactive. Those cells, in which the HDR template is integrated into the genome and the BEC nuclease remains inactive are able to survive the treatment explaining why 100% of the BEC edited cells incorporated the HDR template into their genome.

### Outlook:

Most of the higher organisms (eukaryotes) are able to perform NHEJ. Using genome editing tools, this mechanism can be used to knock out a gene of interest in a rather unspecific way (introduction of indels by the faulty NHEJ repair mechanism). However, as soon as it comes to precise knock-outs or knock-ins using a HDR template, the NHEJ mechanism leads to a lot of technical problems, as part of the edited cells will use the NHEJ pathway to repair their DNA instead of introducing the HDR template thus leading to mixed cell populations with undisired edits. Using BEC family nucleases can overcome this limitation, as BEC nucleases show a novel mode of action to target dsDNA inhibiting the cells' ability to perform NHEJ to survive a DNA double strand break. Furthermore, the cells are forced to introduce the HDR template into their genome to prevent cell death leading to a very efficient way to perform precise gene knock-outs or knock-ins using BEC nucleases.

### Example 4: Construction of an inducible genome editing system for Pichia pastoris (BEC10)

### 4.1 CRISPR/BEC10-Pp-i (inducible) vector system for genome editing in Pichia pastoris

The necessary genetic elements for an inducible expression of the BEC10 nuclease and the constitutive guide RNA (gRNA) transcription were provided in an all-in-one CRISPR/BEC10-Pp-i (inducible) vector system.

### Design of the BEC10 protein expression cassette

The synthetic 3696 bps BEC10 nucleotide sequence was codon optimized for expression in yeast cells, using a bioinformatics application provided by the gene synthesis provider GeneArt (Thermo Fisher Scientific, Regensburg, Germany), SEQ ID NO: 6. Additionally, the DNA nuclease coding sequence was 5' extended by a sequence encoding a SV40 nuclear localization signal (NLS) SEQ ID NO: 17 (Kalderon et al., Cell 39 (1984), 499-509). For protein expression, the resulting synthetic 3723 bps gene was fused to the L-rhamnose-inducible *P. pastoris* LRA4 promoter (SEQ ID NO: 31) (Liu et al., Science Reports 6 (2016), doi: 10.1038/srep27352) and the *P. pastoris* AOX1TT terminator (SEQ ID NO: 19) (Weninger et al., Journal of Biotechnology 235 (2016), 139-149). The final BEC10 protein expression cassette was inserted by Gibson Assembly Cloning (NEB, Frankurt, Germany) into an *E. coli*/*P. pastoris* shuttle vector pPpT6e (EP 3572512 A1), containing all necessary genetic elements for episomal propagation and selection of recombinant *E. coli* and *P. pastoris* cells: For vector propagation and selection of recombinant *E. coli* cells, the plasmid contained the pUC derived high-copy ColE1 origin of replication and the kanMX6 marker gene (SEQ ID NO: 20) under the control of the bifunctional ILV5/synthetic Em72 promoter (SEQ ID NO: 21), (Weninger et al., Journal of Biotechnology 235 (2016), 139-149), conferring kanamycin resistance to *P. pastoris and E. coli* respectively. The Pichia autonomously replicating sequence 1 (PARS1) (SEQ ID NO: 22) allowed episomal replication of the pPpT6e shuttle plasmid in *P. pastoris* cells.

### Design of the tRNA RNA Pol III promoter-mediated gRNA (gRNA) expression cassette

Endogenous *P. pastoris* tRNA genes were used for RNA Pol III promoter-mediated transcription and sequence-specific trimming of tRNA-gRNA fusions to generate functional gRNAs for genome editing (Dalvie et al., ACS Synthetic Biology 9 (2020), 26 -35). For RNA transcription and proper processing, the chimeric gRNA, composed of a constant 19 bps BEC family Stem-Loop Sequence (SEQ ID NO: 14) and the AOX1 target-specific 24 bps spacer sequence (SEQ ID NO: 32), was fused to the native RNA Pol-III promoter/tRNA sequence P_{tRNA1}-tRNA1 (SEQ ID NO: 33) at its 5' end and extended with the native tRNA sequence tRNA4 (SEQ ID NO: 34) at its 3' end. The target spacer sequence was identified in the *P. pastoris* AOX1 coding gene (SEQ ID NO: 35) downstream to the nuclease BEC10 specific PAM motive 5'-TTN-3'. The complete RNA expression cassette (SEQ ID NO: 36) was provided as a synthetic gene fragment by GeneArt (Thermo Fisher Scientific, Regensburg, Germany) and is: 
TTTTGTAGCTTTATAAGCAGCTTTTTCTTGAAGCTTAAGTAGAGTAGTGTGAAAAAGAG
ACCAAGTACAAAATATCCACCTTCTGGGTTTTAGTATTTGTGCTGAGCCGCATGTGTTG
ATAAGAGGCGATAAGGTACAGAAATGATTTGCTAGCAATCCATCATTATTGAAGTGTG
AGTGGGCACCTGATATCATGGCTTGTTTATTCAATAGAGTCTGACATAGTGAAAGGGA
GTTATGAAATTGAAGAAGACTCCATGGCAGGCATATGTAAATATACCAGCCAATCCTA
CTACATTGATCCGTGATAGTTTAGTGGTAGAACCACCGCTTGTCGCGCGGTAGACCGG
GGTTCAATTCCCCGTCGCGGAGCAATTTCTACTGTTGTAGATGGGGATAGCCATCGTTT
CGAATAAGGCAACGTGGCCGAGTGGTTAAGGCGAAAGATTAGAAATCTTTTGGGCTCTG
CCCGCGCAGGTTCGAGTCCTGCCGTTGTCGTCA
Bold: RNA Pol-III promoter/tRNA sequence PtRNA1-tRNA1
Underlined: BEC family Stem-Loop
Italic: AOX1 target-specific spacer sequence
Double underlined: 85 bps tRNA4 sequence

The construction of the all-in-one CRISPR/BEC10-Pp-i (inducible) vector system was completed by cloning the synthetic RNA expression cassette in the prepared *E. coli*/*P. pastoris* pPpT6e shuttle vector, containing the L-rhamnose-inducible BEC10 DNA nuclease expression cassette. The construction of the final CRISPR/BEC10-Pp-i (inducible) vector system was mediated by Gibson Assembly Cloning (NEB, Frankfurt, Germany). The identity of all cloned DNA elements were confirmed by Sanger-Sequencing at LGC Genomics (Berlin, Germany).

### CRISPR/BEC10-Pp-1 (inducible) all-in-one-vector system

The complete nucleotide sequence of the constructed CRISPR/BEC10-Pp-i vector system is provided as SEQ ID NO: 37.

### Design of the Homology-Directed Repair template (HDR)

In order to utilize Homology-Directed Repair (HDR) for gene editing, a synthetic DNA Donor Template (provided by GeneArt (Thermo Fisher Scientific, Regensburg, Germany)) was created encompassing the 720 bps GFP coding sequence flanked by 933 bp left and 1021 right homology arms (SEQ ID NO: 38), to drive reporter gene expression under the controllable Pₐₒₓ₁-promoter.

### 4.2 Pichia pastoris cultivation and transformation

### Preparation of competent P. pastoris CBS7435 cells

Preparation and transformation of competent *P. pastoris* CBS7435 cells were performed as described by Wu & Letchworth, Biotechniques (2014), 36, 152-154. In brief, a single colony of *P. pastoris* was inoculated in 5 ml YPD medium (yeast extract 10 g/L, peptone 20 g/L, 2 % (w/v) glucose) and incubated for 6 to 8 h at 30°C on a horizontal shaker at 250 rpm. Cells of the preculture were diluted into 100 ml YPD medium (10 g/L yeast extract, 20 g/L peptone g/L, 2 % (w/v) glucose) to an optical density at 600 nm (OD600nm) of 0.0025. The inoculated medium was incubated overnight at 30°C on a horizontal shaker at 250 rpm until the culture reached an optical density at OD600nm of 1.0 to 2.0. Cells were harvested by centrifugation for 5 min and 4302 x g at room temperature (RT). The cell density of the culture (cells/mL) was estimated from the optical density at OD600nm according to the following relationship: 1 x OD600nm = 5 × 10⁷ cells/mL. For each transformation, 8 x 10⁸ cells were suspended in 8 mL of 100 mM LiAc, 10 mM DTT, 0.6 M sorbitol and 10 mM Tris-HCI. The resuspended cells were incubated for 30 min at RT. The cells were then pelleted by centrifugation for 5 min and 3999 x g at RT and resuspended in 1.5 mL of ice-cold 1 M sorbitol. The cells were transferred to a 1.5-mL microcentrifuge tube, washed three times with 1.5 mL ice-cold 1 M sorbitol and finally resuspended in 80 µl 1 M icecold sorbitol at a final cell density of about 10¹⁰ cells/mL.

### Transformation of competent P. pastoris cells with the programmed CRISPR/BEC10-Pp-i vector and the HDR template

One aliquot of electrocompetent cells was co-transformed with1 µg of supercoiled CRISPR/BEC10-Pp-i plasmid DNA and additionally with 1.5 µg of linear double-stranded DNA Donor Template in a total volume of 5 µl. The DNA-cell mixture was transferred to a precooled 0.2-cm gap vial and incubated for 5 min on ice. The electroporating pulse was applied at 2 kV, 25 µF, 200 Ω using a Bio-Rad Gene Pulser Xcell electroporation system (Bio-Rad Laboratories, Munich, Germany). The electroporated cells were immediately diluted in 1 mL of ice-cold regeneration medium (0.5 x YPR + 0.5 M sorbitol (1x YPR: 10 g/L yeast extract, 20 g/L peptone, 2 % (w/v) L-rhamnose) sorbitol, to directly induce the BEC10 expression) and transferred into a 2 ml microcentrifuge tube. After incubation for 1 h without agitation and 2 h on a horizontal shaker at 250 rpm (recovery step), transformed cells were diluted in 5 ml YP liquid media containing 2 % (w/v) L-rhamnose (10 g/L yeast extract, 20 g/L peptone , 2 % (w/v) L-rhamnose) and 200 µg/mL geneticin (G418) for plasmid selection. Following growth of cells under selective conditions for at least 3 hours at 30°C on a horizontal shaker at 250 rpm, cells were pelleted by centrifugation and resuspended in 1 mL YPR medium (10 g/L yeast extract, 20 g/L peptone, 2 % (w/v) L-rhamnose). Appropriate cell aliquots were spread on YPR plates (10 g/L yeast extract, 20 g/L peptone, 2 % (w/v) L-rhamnose, 15 g/L agar) supplemented with geneticin (G418) µg/mL and the selective agar plates were incubation for at least 2 days at 30°C.

### Visualizing of CRISPR/BEC10-gRNA mediated Homology-Directed Repair (HDR) using a DNA Donor Template that expresses green fluorescence protein (GFP)

The frequency of HDR-edited *P. pastoris* cells was visualized by co-transforming competent cells with a GFP-expressing DNA Donor Template and with a CRISPR/BEC10-Pp-i plasmid (SEQ ID NO: 37), targeting the genomic AOX1 locus with the plasmid-expressed gRNA (SEQ ID NO: 36). This approach enabled screening of transformed cell population for CRISPR/BEC10-directed gene integration into the strictly regulated methanol inducible AOX1 locus. AOX1 gene disruption and site-specific GFP-reporter gene integration in recombinant *P. pastoris* cells was detected using a colony filter assay which was carried out as follows: After cell recovery and BEC counterselection in L-rhamnose-containing medium, transformed cells were plated on NC transfer membrane filters (Merck Chemicals, Darmstadt, Germany) applied directly to the surface of YPR agar plates (10 g/L yeast extract, 20 g/L peptone g/L, 2 % (w/v) L-rhamnose, 15 g/L agar) supplemented with 200 µg/mL geneticin. After incubation for at least 2 days at 30 °C, the filters with grown cells were transferred onto selective YP agar plates (10 g/L yeast extract, 20 g/L peptone g/L, 15 g/L)) supplemented with 2 % (v/v) methanol (YPM) and 200 µg/mL geneticin (G418) to induce Pₐₒₓ₁-driven GFP expression. After another two days of incubation at 30 °C, GFP expressing colonies were detected under UV light, using the Fusion fx7 chemiluminescence imaging system (Vilber, Erberhardzell, Germany).

### Confirmation of site-specific integration of DNA Donor Template in the targeted AOX1 locus

Site specific integration of the 2694 bps GFP-encoding DNA Donor Template in the targeted AOX1 locus of GFP-expressing *P. pastoris* cells was confirmed by diagnostic PCR on chemical lysed cells as template (Colony-PCR) using primers PCR_AOX1_fw (SEQ ID NO: 39) and PCR_AOX1_rv (SEQ ID NO: 40). The designed oligonucleotides, that did not bind within the DNA Donor Template, produced a PCR product of 2224 bps on genomic DNA from P. pastoris wild-type cells and a PCR product of 2924 bps on genomic DNA from *P. pastoris* GFP::AOX1 mutant clones.

### Varying the timing of induced conditions for BEC10 expression after the transfection step

To investigate the effect of a delayed BEC10 expression, the standard transformation protocol, as given above, was modified by varying the timing of L-rhamnose-induced BEC10 nuclease expression after DNA transfection by electroporation. The frequency of successful genome editing was analyzed by inducing plasmid-based BEC10 expression immediately after the pulsing as well as three and six hours after the electroporation step. Thus, the BEC-mediated counter-selection of wild-type cells was delayed and the transformed cells were given more time for homologous recombination of the DNA Donor Template fragment. For the experiments carried out, competent *P. pastoris* CBS7435 Δdnl4 cells were used, which predominantly support integration of homologous DNA fragments by the homologous recombination (HR) process (Cai et al. Nucleic Acids Research 49 (2021), 7791-7805). Preparation and electroporation procedure of competent cells was done as described in the standard protocol above. For varying the conditions for induced BEC10 expression the culture medium composition for cell recovery and selective passage in liquid medium before plating were modified as summarized in the table below.

| Experiment | Liquid Medium for Cell recovery after transfection [3 h] | Liquid Medium for selective cultivation [3 h] | Agar growth plate for clone selection [≥ 2 d] | Induction of the BEC10 expression |
|---|---|---|---|---|
| **A** | YP**R**-Sorbitol | YP**R**-G418 | YP**R**-G418 | immediately |
| **B** | YP**D**-Sorbitol | YP**R**-G418 | YP**R**-G418 | After 3 h |
| **C** | YP**D**-Sorbitol | YP**D**-G418 | YP**R**-G418 | After 6 h |

| | | | | |
|---|---|---|---|---|
| YPR-Sorbitol: 0.5 × YPR, 0.5 M sorbitol liquid medium, containing 1% (w/v) L-rhamnose YPD-Sorbitol: 0.5 × YPD, 0.5 M sorbitol liquid medium, containing 1% (w/v) glucose YPR-G418: Selective medium, containing 2 % (w/v) L-rhamnose and 200 µg/ml geneticin YPD-G418: Selective medium, containing 2 % (w/v) glucose and 200 µg/ml geneticin | | | | |

All transformation experiments A, B and C were performed in duplicates. Frequency of site-specific genomic integration of the transformed DNA Donor Template fragment in *P. pastoris* was performed as described above: Transformed cells were spread on NC transfer membrane filters (Merck Chemicals, Darmstadt, Germany) applied directly to the surface of YPR agar plates supplemented with 200 µg/ml geneticin. Subsequently filters with grown cells were transferred on selective YPM-plates for induced GFP-expression conditions. For detection of fluorescence signals emitted by GFP proteins under UV light, the Fusion fx7 chemiluminescence imaging system (Vilber, Erberhardzell, Germany) was used.

### Example 5: Comparison of the BEC induced genome-editing efficiency using a delayed nucleases expression

The example demonstrates the usage of an optimized approach to apply BEC family nucleases for efficient genome-editing. As described in the above examples, BEC family nucleases are a new type of class 2 nucleases with a novel mechanism (collateral activity) to target DNA & RNA, which can be used for genome editing in combination with a homology directed repair template.

To demonstrate the advantage of our newly developed approach to enhance the genome editing efficiency of the BEC family nucleases experiments were carried out using a vector system allowing for inducible expression of the BEC10 nuclease.

### Experimental Setup:

In this example, the CRISPR/BEC10-Pp-i vector system and a HDR template were used to knock-in a GFP gene into the AOX locus of *P. pastoris.* Three parallel approaches where carried out where the expression of the BEC was initiated at three different time points.
A: BEC expression immediately after the transformation
B: BEC expression 3h after the transformation
C: BEC expression 6 h after the transformation
In addition to that, negative control experiments performed using a spacer sequence that does not match the genome of *P. pastoris.*

The cells were transformed, cultivated and plated as described in 4.2. The culture plates were incubated as described in 4.2 and afterwards analyzed by counting the number of grown colonies and by the evaluation of their phenotype (GFP expression) and genotype (diagnostic PCR).

### Results:

All experiments were carried out in two biological replicates and the results obtained from these replicates were combined to evaluate the genome editing activity depending on the timing of the BEC10 induction (Exemplary plates are shown in Figure 4).

In the experimental setup the CRISPR/BEC10-Pp-i expression construct was transformed into *P. pastoris* cells together with the HDR template and the BEC expression was induced: A: instantly, B: 3 h after transformation, C: 6 h after transformation.
A: In the experimental approach where the BEC10 expression was induced instantly after the transformation, the evaluated plates showed on average 24 grown colonies with 16 colonies showing a GFP positive phenotype (66.6%). In contrast to this, the negative control experiments showed on average > 5.000 grown colonies with less than 0.1% of the colonies showing a GFP positive phenotype.
B: In the experimental approach where the BEC10 expression was induced 3 h after the transformation, the evaluated plates showed on average 90 grown colonies with 81 colonies showing a GFP positive phenotype (90.0%). In contrast to this, the negative control experiments showed on average > 5000 grown colonies with less than 0.1% of the colonies showing a GFP positive phenotype.
C: In the experimental approach where the BEC10 expression was induced 6 h after the transformation, the evaluated plates showed on average 264 grown colonies with 241 colonies showing a GFP positive phenotype (91.3%). In contrast to this, the negative control experiments showed on average > 5000 grown colonies with less than 0.1% of the colonies showing a GFP positive phenotype.

To validate the correct and precise integration of the HDR Template into the AOX locus, 25 randomly selected GFP positive colonies were selected and analyzed like described in 4.2. This experimental approach confirmed the correct integration of the HDR template in 25 out of 25 colonies (100%).

In summary these results demonstrate that a decoupling of the transformation of HDR template and the expression of the BEC nuclease can increase the genome editing efficiency and the overall colony number of successfully edited cells dramatically.

### Conclusion:

The generated results are showing an efficient way to enhance the genome editing efficiency of BEC family nucleases. In contrast to classical CRISPR nucleases where the activation of the nuclease is leading to a guided double strand break on a predefined position in the DNA, BEC family nucleases induces collateral DNA & RNA degradation after an initial, guide specific activation leading to cell death (guided toxicity). Even though, this guided toxicity mechanism is far away from the DNA targeting mechanism of classical CRISPR nucleases it has already been shown that BEC family nucleases can be used to perform genome editing when combined with a HDR template. This genome editing events can be explained by the fact, that BEC family nucleases, once activated by specific matching of the spacer and protospacer sequences, initiate collateral DNA and RNA degradation leading to cell death. However, before the BEC nuclease is activated some cells will introduce the co-transformed HDR template into their genome by naturally occurring homologous recombination, leading to an altered PAM and protospacer sequence within the genome. Therefore, the protospacer sequence matching the spacer sequence of the BEC gRNA is no longer present in the genome and the BEC nuclease stays inactive. Consequently, all cells without a correct integration of the HDR template are depleted. To enhance the genome editing efficiency of BEC family nucleases, the time course of homologous integration of the HDR template was decoupled from the expression of the BEC nuclease. By doing so, the cells are provided with more time to natively integrate the HDR template into their genome before the BEC is expressed and depletes all cells without a proper integration. This advantageously leads to significantly higher genome editing efficiency and enrichment of genome modified clones.

## Claims

1. A method for the genome editing of cells at a target locus by homology directed repair (HDR) within a cell population and concurrently enriching within said cell population the HDR-genome edited cells, wherein the method comprises
(A) introducing into the cells within the cell population one or more nucleic acid molecules, said one or more nucleic acid molecules encoding in expressible form
(i) a CRISPR nuclease comprising or consisting of
(a) an amino acid sequence of any one of SEQ ID NO: 1, 2 or 3;
(b) an amino acid sequence being encoded by the nucleotide sequence of SEQ ID NO: 4, 5, 6 or 7;
(c) an amino acid sequence being at least 80% identical to the amino acid sequence of (a), or
(d) an amino acid sequence being encoded by a nucleic acid sequence being at least 80 % identical to the nucleotide sequence of (b),
(ii) a guide RNA comprising (a) a first segment comprising a nucleotide sequence that is complementary to a sequence at the target locus and (b) a second segment that interacts with the CRISPR nuclease of (i),
wherein the CRISPR nuclease and/or the guide RNA are encoded by said one or more nucleic acid molecules in a form wherein the expression can be induced or repressed, and
a nucleic acid molecule comprising, consisting of, or encoding
(iii) a donor template with homology to the target locus; and
(B) culturing the cells at least for a time wherein the cells double under growth conditions wherein the target locus within the cells of the cell population becomes modified by homology directed repair (HDR), and
(C) inducing after step (B) or repressing in steps (A) and (B) the expression of the CRISPR nuclease and/or the guide RNA within the cells thereby enriching within said cell population the HDR-genome edited cells.

2. The method of claim 1, wherein in the form wherein the expression can be repressed the expression of the CRISPR nuclease and/or the guide RNA is under the control of an inducible promoter, preferably a chemically inducible promoter and/or wherein in the form wherein the expression can be repressed the expression of the CRISPR nuclease and/or the guide RNA is under the control of a repressible promoter, preferably a chemically repressible promoter.

3. A method for the genome editing of cells at a target locus by homology directed repair (HDR) within a cell population and concurrently enriching within said cell population the HDR-genome edited cells, wherein the method comprises
(A) introducing into the cells within the cell population
a nucleic acid molecule, said nucleic acid molecule encoding in expressible form
(i) a guide RNA comprising (a) a first segment comprising a nucleotide sequence that is complementary to a sequence at the target locus and (b) a second segment that interacts with the CRISPR nuclease of (C), and
a nucleic acid molecule comprising, consisting of, or encoding
(ii) a donor template with homology to the target locus;
(B) culturing the cells at least for a time wherein the cells double under growth conditions wherein the target locus within the cells of the cell population becomes modified by homology directed repair (HDR), and
(C) introducing after (B) into the cells within the cell population a CRISPR nuclease comprising or consisting of
(a) an amino acid sequence of any one of SEQ ID NO: 1, 2 or 3;
(b) an amino acid sequence being encoded by the nucleotide sequence of SEQ ID NO: 4, 5, 6 or 7;
(c) an amino acid sequence being at least 80% identical to the amino acid sequence of (a), or
(d) an amino acid sequence being encoded by a nucleic acid sequence being at least 80 % identical to the nucleotide sequence of (b),
thereby enriching within said cell population the HDR-genome edited cells.

4. A method for the genome editing of cells at a target locus by homology directed repair (HDR) within a cell population and concurrently enriching within said cell population the HDR-genome edited cells, wherein the method comprises
(A) introducing into the cells within the cell population
a nucleic acid molecule, said nucleic acid molecule encoding in expressible form
(i) a CRISPR nuclease comprising or consisting of
(a) an amino acid sequence of any one of SEQ ID NO: 1, 2 or 3;
(b) an amino acid sequence being encoded by the nucleotide sequence of SEQ ID NO: 4, 5, 6 or 7;
(c) an amino acid sequence being at least 80% identical to the amino acid sequence of (a), or
(d) an amino acid sequence being encoded by a nucleic acid sequence being at least 80 % identical to the nucleotide sequence of (b), and
a nucleic acid molecule comprising, consisting of, or encoding
(ii) a donor template with homology to the target locus;
(B) culturing the cells at least for a time wherein the cells double under growth conditions wherein the target locus within the cells of the cell population becomes modified by homology directed repair (HDR), and
(C) introducing after (B) into the cells within the cell population a guide RNA comprising (a) a first segment comprising a nucleotide sequence that is complementary to a sequence at the target locus and (b) a second segment that interacts with the CRISPR nuclease of A (i),
thereby enriching within said cell population the HDR-genome edited cells.

5. A method for the genome editing of cells at a target locus by homology directed repair (HDR) within a cell population and concurrently enriching within said cell population the HDR-genome edited cells, wherein the method comprises
(A) introducing into the cells within the cell population
a nucleic acid molecule comprising, consisting of, or encoding a nucleic acid molecule encoding in expressible form a donor template with homology to the target locus; and
(B) culturing the cells at least for a time wherein the cells double under growth conditions wherein the target locus within the cells of the cell population becomes modified by homology directed repair (HDR), and
(C) introducing after (B) into the cells within the cell population
a ribonucleoprotein complex (RNP) comprising or consisting of
a CRISPR nuclease comprising or consisting of
(a) an amino acid sequence of any one of SEQ ID NO: 1, 2 or 3;
(b) an amino acid sequence being encoded by the nucleotide sequence of SEQ ID NO: 4, 5, 6 or 7;
(c) an amino acid sequence being at least 80% identical to the amino acid sequence of (a), or
(d) an amino acid sequence being encoded by a nucleic acid sequence being at least 80 % identical to the nucleotide sequence of (b),
in complex with a guide RNA comprising (a) a first segment comprising a nucleotide sequence that is complementary to a sequence at the target locus and (b) a second segment that interacts with the CRISPR nuclease;
thereby enriching within said cell population the HDR-genome edited cells.

6. A method for the genome editing of cells at a target locus by homology directed repair (HDR) within a cell population and concurrently enriching within said cell population the HDR-genome edited cells, wherein the method comprises
(A) introducing into the cells within the cell population a nucleic acid molecule comprising, consisting of, or encoding a nucleic acid molecule encoding in expressible form a donor template with homology to the target locus; and
(B) culturing the cells at least for a time wherein the cells double under growth conditions wherein the target locus within the cells of the cell population becomes modified by homology directed repair (HDR), and
(C) introducing after (B) into the cells within the cell population
(i) a CRISPR nuclease comprising or consisting of
(a) an amino acid sequence of any one of SEQ ID NO: 1, 2 or 3;
(b) an amino acid sequence being encoded by the nucleotide sequence of SEQ ID NO: 4, 5, 6 or 7;
(c) an amino acid sequence being at least 80% identical to the amino acid sequence of (a), or
(d) an amino acid sequence being encoded by a nucleic acid sequence being at least 80 % identical to the nucleotide sequence of (b), or
(ii) a nucleic acid molecule encoding the CRISPR nuclease of (i) in expressible form, and
in addition a guide RNA comprising (a) a first segment comprising a nucleotide sequence that is complementary to a sequence at the target locus and (b) a second segment that interacts with the CRISPR nuclease or a nucleic caid in expressible;
thereby enriching within said cell population the HDR-genome edited cells.

7. A method for the genome editing of cells at a target locus by homology directed repair (HDR) within a cell population and concurrently enriching within said cell population the HDR-genome edited cells, wherein the method comprises
(A) introducing into the cells within the cell population a nucleic acid molecule encoding in expressible form
(i) a guide RNA comprising (a) a first segment comprising a nucleotide sequence that is complementary to a sequence at the target locus and (b) a second segment that interacts with the CRISPR nuclease of (C), and
a nucleic acid molecule comprising, consisting of, or encoding
(ii) a donor template with homology to the target locus; and
(B) culturing the cells at least for a time wherein the cells double under growth conditions wherein the target locus within the cells of the cell population becomes modified by homology directed repair (HDR), and
(C) introducing after (B) into the cells within the cell population a nucleic acid molecule encoding in expressible form
a CRISPR nuclease comprising or consisting of
(a) an amino acid sequence of any one of SEQ ID NO: 1, 2 or 3;
(b) an amino acid sequence being encoded by the nucleotide sequence of SEQ ID NO: 4, 5, 6 or 7;
(c) an amino acid sequence being at least 80% identical to the amino acid sequence of (a), or
(d) an amino acid sequence being encoded by a nucleic acid sequence being at least 80 % identical to the nucleotide sequence of (b),
thereby enriching within said cell population the HDR-genome edited cells.

8. A method for the genome editing of cells at a target locus by homology directed repair (HDR) within a cell population and concurrently enriching within said cell population the HDR-genome edited cells, wherein the method comprises
(A) introducing into the cells within the cell population a nucleic acid molecule encoding in expressible form
(i) a CRISPR nuclease comprising or consisting of
(a) an amino acid sequence of any one of SEQ ID NO: 1, 2 or 3;
(b) an amino acid sequence being encoded by the nucleotide sequence of SEQ ID NO: 4, 5, 6 or 7;
(c) an amino acid sequence being at least 80% identical to the amino acid sequence of (a), or
(d) an amino acid sequence being encoded by a nucleic acid sequence being at least 80 % identical to the nucleotide sequence of (b), and
a nucleic acid molecule comprising, consisting of, or encoding
(ii) a donor template with homology to the target locus; and
(B) culturing the cells at least for a time wherein the cells double under growth conditions wherein the target locus within the cells of the cell population becomes modified by homology directed repair (HDR), and
(C) introducing after (B) into the cells within the cell population a nucleic acid molecule encoding in expressible form
a guide RNA comprising (a) a first segment comprising a nucleotide sequence that is complementary to a sequence at the target locus and (b)
a second segment that interacts with the CRISPR nuclease of (A)(i)
thereby enriching within said cell population the HDR-genome edited cells.

9. The method of any one of claims 1 to 8, wherein the method further comprises (D) isolating after (C) one or more cells, wherein the target locus has been genome edited by homology directed repair (HDR).

10. The method of any one of claims 1 to 9, wherein the donor template (i) introduces one or more mutations at the target locus, (ii) template inserts one or more nucleotides at the target locus, (iii) deletes one or more nucleotides at the target locus and/or (iv) substitutes one or more nucleotides at the target locus.

11. The method of any one of claims 1 to 10, wherein the cells are cultured in step (B) at least for a time wherein the cells double 1.5 times, preferably 2 times, more preferably 2.5 times and most preferably 3.0 times.

12. The method of any one of claims 1 to 11, wherein the donor template comprises or consists of a nucleotide sequence with one or more intended mutations flanked by nucleotide sequences being homologous to the target locus.

13. The method of any one of claims 1 to 12, wherein the cells within the population are capable of repairing double-stranded DNA breaks through non-homologous end joining (NHEJ).

14. One or more vector(s) comprising in expressible form
(i) a CRISPR nuclease comprising or consisting of
(a) an amino acid sequence of any one of SEQ ID NO: 1, 2 or 3;
(b) an amino acid sequence being encoded by the nucleotide sequence of SEQ ID NO: 4, 5, 6 or 7;
(c) an amino acid sequence being at least 80% identical to the amino acid sequence of (a), or
(d) an amino acid sequence being encoded by a nucleic acid sequence being at least 80 % identical to the nucleotide sequence of (b),
(ii) a guide RNA comprising (a) a first segment comprising a nucleotide sequence that is complementary to a sequence at the target locus; and (a) a second segment that interacts with the CRISPR nuclease of (i),
wherein the CRISPR nuclease and/or the guide RNA are encoded by said one or more nucleic acid molecules in a form wherein the expression can be induced or repressed, and
(iii) optionally a donor template with homology to the target locus.

15. The method of any one of claims 1 to 13 or the one or more vector(s) of claim 14, wherein the sequence identity of at least 80% is at least 85%, preferably at least 90% and most preferably at least 95%.
